# DEMANDE DE BREVET EUROPEEN

(11) **EP 1 672 072 A2**
(43) Date de publication de la demande: **21.06.2006**
(21) Numéro de dépôt: 05025860.7
(22) Date de dépôt: 28.06.1996
(51) Int. Cl.: C12N 15/31, C12N 15/01, C07K 14/205, C12N 1/20, G01N 33/569, G01N 33/577, A61K 39/106, A61K 39/40, C12Q 1/10, C12R 1/01, C12N 9/80

(54) **Clonage et caractérisation du gène flbA de H. pylori; production de souches aflagellées**

(30) Priorité: 04.07.1995 FR 9508068
(62) Demande divisionnaire de: 96401445.0
(71) Demandeur: INSTITUT PASTEUR, 75724 Paris Cédex 15 (FR); INSTITUT NATIONAL DE LA SANTE ET DE LA RECHERCHE MEDICALE (INSERM), 75654 Paris Cédex 13 (FR)
(72) Inventeur: Suerbaum, Sebastian, 44787 Bochum (DE); Labigne, Agnès, 91440 Bures Sur Yvette (FR)
(74) Mandataire: Desaix, Anne

(57) **Abrégé**

La présente demande concerne des séquences nucléotidiques régulant la biosynthèse des protéines des flagelles d'Helicobacter pylori, les protéines codées par ces séquences ainsi que des souches bactériennes aflagellées. L'invention vise aussi l'utilisation de ces moyens pour la détection d'une infection par H. pylori ou pour la protection contre une telle infection.

## Description

Helicobacter pylori (désigné également par l'expression H. Pylori) est une bactérie à gram négatif retrouvée exclusivement à ce jour à la surface de la muqueuse de l'estomac chez l'homme.

Comme la plupart des bactéries, H. Pylori est sensible à un milieu de pH acide mais cependant peut tolérer l'acidité en présence de taux physiologiques d'urée (Marshall et al (1990) Gastroenterol. 99: 697-702). En hydrolysant furée sous forme de dioxyde de carbone et d'ammoniac qui sont relargués dans le microenvironnement de la bactérie, l'uréase de H. pylori permet la survie de la bactérie dans l'environnement acide de l'estomac. Récemment des études menées sur des modèles animaux ont fourni des éléments suggérant que l'uréase est un facteur important dans la colonisation de la muqueuse gastrique (Eaton et al (1991) Infect. Immun. 59: 2470-2475). L'uréase est également suspectée de causer des dommages soit directement, soit indirectement, à la muqueuse gastrique.

Helicobacter pylori (H. pylori) est à l'heure actuelle reconnu comme l'agent étiologique des gastrites antrales, et apparaît comme un des cofacteurs requis pour le développement des ulcères. Par ailleurs il semble que le développement de carcinomes gastriques puisse être associé à la présence de H. pylori

Afin de mettre au point des moyens nouveaux sensibles et spécifiques pour la détection in vitro d'infections par des bactéries de l'espèce Helicobacter pylori, les inventeurs se sont intéressés au système de régulation de la mobilité de ces bactéries.

Dans ce but, ils se sont intéressés à différentes modifications des souches de H. pylori. modifications qui n'affectaient pas la reconnaissance de ces bactéries par des sérums de patients infectés mais qui néanmoins permettaient de ne pas obtenir de réaction de type « faux positif» en particulier avec des bactéries de la famille de Campylobacter et par exemple, Campylobacter jejuni.

Les inventeurs ont en outre constaté que les bactéries modifiées obtenues pouvaient être, le cas échéant, utilisées dans le cadre de la fabrication de compositions immunogènes ou vaccinantes. A cet égard, l'invention propose notamment des souches bactériennes vivantes atténuées.

Dans une première étape, les inventeurs ont identifié et isolé le gène fIbA ayant une implication dans la régulation de la biosynthèse des flagelles de H. pylori et par conséquent, dans la régulation de la mobilité de la bactérie. La biosynthèse des flagelles comprend la synthèse des flagellines A et B, et de la gaine. Le gène fIbA régule à la fois la synthèse des flagellines A et B, et la synthèse de la gaine contenant ces flagellines. Les inventeurs ont établi que le gène fIbA était également intéressant en ce qu'il régulait la biosynthèse de la protéine d'ancrage de la bactérie, encore appelée "crochet".

L'invention a donc pour objet une séquence nucléotidique du gène flbA régulant la biosynthèse des protéines des flagelles d'Helicobacter pylori, caractérisée en ce qu'elle est capable d'hybrider dans des conditions de forte stringence avec une sonde correspondant à un fragment de nucléotides de H. pylori amplifié à l'aide de deux oligonuctéotides répondant aux séquences suivantes :
OLFIbA-1 : ATGCCTCGAGGTCGAAAAGCAAGATG
OLFIbA-2 : GAAATCTTCATACTGGCAGCTCCAGTC, ou capable d'hybrider dans des conditions de forte stringence avec ces oligonucléotides.
Une telle séquence peut être obtenue par les étapes de :
- criblage d'une banque génomique contenant l'ADN chromosomique d'une souche H. pylori avec une sonde correspondant à un fragment de nucléotides de H. pylori amplifié à l'aide de deux oligonucléotides répondant aux séquences suivantes :
   OLFIbA-1 : ATGCCTCGAGGTCGAAAAGCAAGATG
   OLFIbA-2 : GAAATCTTCATACTGGCAGCTCCAGTC, ou capable d'hybrider dans des conditions de forte stringence avec ces oligonucléotides.
- récupération des séquences d'ADN hybridant avec ladite sonde,
- sous-clonage des séquences d'ADN obtenues dans un vecteur approprié de type plasmide et sélection de ceux des vecteurs modifiés hybridant dans des conditions de forte stringence avec la sonde correspondant au fragment d'ADN de H. pylori amplifié à l'aide des oligonucléotides OLFIbA-1 et OLFIbA-2,
- séquençage des fragments d'ADN contenus dans les vecteurs plasmidiques hybridant avec la susdite sonde et détermination de la phase ouverte de lecture contenue dans ces fragments.

Avantageusement, on utilisera ces fragments d'ADN pour reconstituer la séquence codante du gène flbA correspondant à une phase ouverte de lecture comportant environ 2196 nucléotides.

La banque génomique contenant l'ADN chromosomique de H. pylori peut être obtenue à partir de toute souche de H. pylori. On peut aussi préparer une banque cosmidique à partir de l'ADN chromosomique de H. pylori.

Une souche utilisable pour la réalisation de cette banque est par exemple la souche N6 déposée à la NCIMB le 26 Juin 1992, sous le n° NCIMB40512.

Les deux oligonucléotides amorces utilisés pour préparer la sonde destinée à l'hybridation de l'ADN recherché présent dans la banque d'ADN de H. pylori sont choisis parmi les régions conservées des différentes protéines de la famille LcrD/FlbF.

Les deux oligonucléotides amorces OLFIbA-1 et OLFlbA-2 on permis d'amplifier un fragment, utilisable comme sonde, de 130 paires de bases répondant à la séquence suivante :

ATG CCA GGA AAG CAA ATG GCG ATT GAT GCG GAT TTA AAT TCA
GG4 CTT ATT GAT GAT AAG GAA GCT AAA AAA CGG CGC GCC GCT
CTA AGC CAA GAA GCG GAT TTT TAT GGT GCG ATG GAT GGC GCG
TCT AAA TTT

Les conditions de forte stringence dont il est question ci-dessus sont les suivantes : l'hybridation est réalisée à 42°C en présence de 50% Formamide dans un tampon 2XSSC contenant 0,1% SDS (1XSSC correspond à 0,15 M NaCl plus 15 mM de citrate de sodium - pH 7,0), Les lavages sont réalisés avec 2XSSC plus 0,1% SDS à 68°C par exemple deux fois pendant une heure.

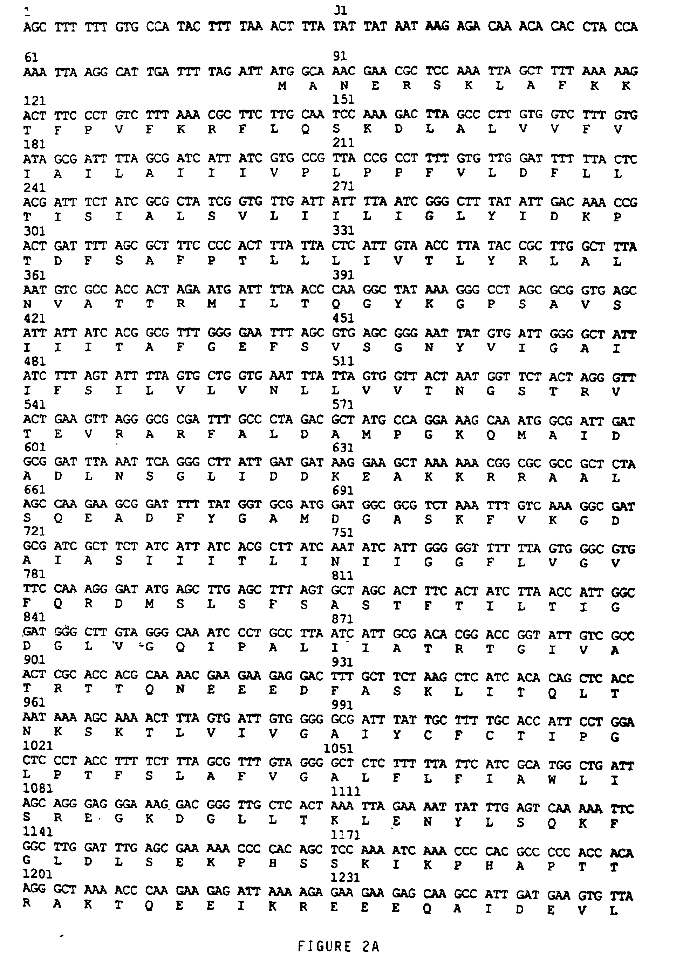

Une séquence nucléotidique particulièrement intéressante selon l'invention est la séquence du gène flbA correspondant à l'enchaînement de nucléotides représenté à la figure 2, ou à un enchaînement nucléotidique hybridant dans des conditions de forte stringence avec le susdit enchaînement.

Selon un autre mode de réalisation de l'invention, la séquence nucléotidique, objet de la présente demande, est caractérisée en ce qu'elle code pour une protéine répondant à la séquence d'acides aminés représentée à la figure 2 ou pour une séquence d'acides aminés ayant les mêmes propriétés régulatrices vis à vis de la biosynthèse des protéines des flagelles de H. pylori, que la susdite séquence.

L'invention a également pour objet une séquence nucléotidique répondant aux définitions précédentes, modifiée par délétion, substitution ou insertion de bases ou d'un fragment d'une séquence de nucléotides, de façon telle que :
- soit le gène fIbA n'est plus exprimé dans un hôte cellulaire,
- soit l'expression du gène flbA dans un hôte cellulaire ne permet pas la biosynthèse des flagellines A et B ni de la gaine les contenant et le cas échéant ne permet pas la synthèse de la protéine d'ancrage de H. pylori ou le crochet.

La modification subie par la séquence nucléotidique de l'invention doit être telle qu'elle est irréversible et notamment qu'elle reste irréversible, lors de la recombinaison de cette séquence avec le gène fIbA présent dans une bactérie que l'on transformerait avec une séquence nucléotidique ainsi modifiée. Cette recombinaison est par exemple de type « double crossing over ». De préférence, la modification de la séquence nucléotidique ne doit pas entraîner de modification substantielle - après remplacement par cette séquence modifiée du fragment correspondant du gène fIbA normal dans une souche déterminée de H. pylori - des fonctions des gènes voisins.

Entrent également dans le cadre de l'invention, des séquences nucléotidiques constituant un fragment du gène fIbA répondant aux critères précédents. A titre d'exemples, des fragments objet de l'invention, comportent au moins 6 nucléotides, de préférence au moins 50, voire au moins 100 nucléotides.

De tels fragments sont par exemple choisis soit pour leur caractère spécifique du gène flbA, soit pour leur appartenance à des régions conservées de plusieurs gènes codant pour des protéines de la famille LcrD/FlbF.

Selon un autre mode de réalisation, l'invention vise également les fragments du gène flbA limités par les sites de restriction présents sur le gène. Certains de ces sites sont définis à titre d'exemple à la figure 1 B.

Un autre fragment selon l'invention est un fragment d'au moins 1000 pb issu d'une quelconque région du gène flbA et comprenant de préférence un site de restriction ou susceptible d'accueillir un site de restriction.

D'autres séquences nucléotidiques de l'invention sont par exemple des acides nucléiques recombinants comprenant une séquence nucléotidique telle que celles qui ont été décrites ci-dessus, elle-même modifiée par insertion d'une cassette contenant un marqueur, par exemple un gène de résistance à un antibiotique ou un gène de résistance à un métal lourd tel que décrit dans la demande FR 9406202 déposée le 20/05/94.

Une insertion peut ainsi être effectuée avec une cassette de résistance à la kanamycine. A cet égard, différentes techniques peuvent être mises en oeuvre et on se réferrera notamment à la publication de Labigne A. et al (J. of Bacteriology, vol. 170, 1988, p. 1704-1708) et à la publication de Labigne A. et al (Res. Microbiol 1992, 143, 15-26).

L'invention a également pour objet des oligonucléotides spécifiques d'une séquence nucléotidique déterminée précédemment et caractérisés en ce qu'ils répondent à l'une des séquences suivantes :
OLFIbA-1: ATGCCTCGAGGTCGAAAAGCAAGATG
OLFIbA-2: GAAATCTTCATACTGGCAGCTCCAGTC
OLFIbA-7: CGGGATCCGTGGTTACTAATGGTTCTAC
OLFIbA-8: CGGGATCCTCATGGCCTCTTCAGAGACC

Selon une autre variante de réalisation, l'invention concerne une séquence d'acides aminés de la protéine FIbA de H. pylori caractérisée en ce qu'elle est codée par une séquence nucléotidique telle que déterminée précédemment.

Une séquence d'acides aminés particulière de la protéine FlbA de H. pylori est présentée à la figure 2.

Ainsi, dans le cadre de l'invention, le gène flbA et la protéine exprimée par ce gène peuvent présenter un intérêt, en particulier pour l'utilisation dans des compositions immunogènes ou vaccinantes.

L'invention vise également des souches bactériennes d'Helicobacter pylori ayant un phénotype aflagellé résultant de la mutation par substitution, addition et/ou de la délétion de bases ou d'un fragment nucléotidique de la séquence nucléotidique définie ci-dessus du gène flbA participant à la régulation de la biosynthèse des protéines des flagelles de H pylori.

Cette modification du gène fIbA permet l'obtention d'une souche de type aflagellé c'est à dire n'exprimant plus les protéines FIaA et FIaB et de préférence n'exprimant plus les protéines de la gaine.

Selon une variante de réalisation de cette souche bactérienne, la souche obtenue sera en outre dépourvue de la protéine du crochet de H. pylori.

De façon préférée, une souche bactérienne répondant aux critères précédents est caractérisée en ce qu'elle est obtenue à partir de la souche N6 déposée à la NCIMB le 26 Juin 1992 sous le numéro NCIMB 40512.

A titre d'exemple, l'invention a pour objet une souche aflagellée recombinante de H. pylori, désignée par N6flbA- et déposée à la NCIMB le 30 Juin 1995 sous le n° NCIMB 40747.

De telles souches aflagellées de H. pylori sont particulièrement intéressantes pour des utilisations en sérologie et par conséquent pour la détection in vitro d'une infection par H. pylori. Avantageusement, ces souches sont de type recombinantes.

Ces souches présentent notamment l'avantage de permettre une détection in vitro spécifique et sensible de l'infection par H. pylori. En d'autres termes, l'invention permet avantageusement de détecter spécifiquement une infection par H. pylori en évitant en particulier les résultats « faux positifs » par exemple avec des souches de bactéries telles que Salmonella ou Campylobacter..

Etant donné que les souches de H. pylori de type aflagellé ainsi définies peuvent avoir également d'autres applications, et par exemple être mises en oeuvre dans le cadre de la préparation de compositions de vaccins, on pourra avoir intérêt à préparer des souches bactériennes recombinantes aflagellées ayant une seconde modification ou mutation et par exemple on pourra avoir recours à une souche bactérienne aflagellée caractérisée en ce qu'elle est en outre mutée de façon à produire une uréase atténuée, voire à ne plus produire d'uréase, la mutation consistant par exemple en une mutation de la séquence nucléotidique d'un ou plusieurs gènes choisis parmi les gènes ureA, ureB, ureC, ureD, ureE, uref, ureG, ureH ou urel. Les gènes de structure de l'uréase désignés par ureA, ureB, ureC et ureD de l'uréase ont été décrits dans la publication (Labigne et al (1991) J. Bacteriol. 173: 1920-1931). Les autres gènes ont été décrits dans la demande de brevet EP 0610322.

Les souches bactériennes de l'invention peuvent être utilisées telles quelles ou sous la forme d'extrait et en particulier l'invention a pour objet un extrait bactérien total de souche obtenu à partir des souches précédemment décrites.

Un tel extrait bactérien peut être préparé par extraction au N-OctylGlucoside. En ce cas, la technique de préparation utilisée est celle décrite par LELWALA-GURUGE J. (Scand. J. Infect. Dis. 1992, 24: 457-465).

Un autre extrait bactérien peut être obtenu par extraction au PBS ou la glycine selon les techniques respectivement décrites par BAZILLOU M. et al (Clin. Diagn. Lab. Immuno., 1994, 1: 310-317) et AGUIRRE P.M. (Eur. J. Clin. Microbiol. Infect. Dis., 1992, 11: 634-639).

Dans le cadre de ces applications, l'invention concerne une composition pour la détection in vitro d'une infection par H. Pylori dans un échantillon de fluide biologique chez un patient, notamment dans un échantillon de sérum, comprenant à titre de principe actif, une souche bactérienne de l'invention ou un extrait bactérien selon la description donnée ci-dessus.

Les échantillons biologiques utilisés peuvent être de tous types et en particulier, il peut s'agir de tous fluides biologiques comme par exemple le sérum, la salive ou l'urine.

De même, les techniques mises en oeuvre pour la détection sont toutes les techniques faisant intervenir des réactions de type immunologique et en particulier de type antigène-anticorps. On utilise par exemple les techniques de type Western Blot, ELISA...

L'invention concerne donc également un procédé de détection in vitro d'une infection par H. pylori dans un échantillon de fluide biologique chez un patient, notamment dans un échantillon de sérum, comprenant les étapes de :
- mise en contact de l'échantillon testé avec une souche bactérienne selon l'invention ou un extrait bactérien défini plus haut,
- détection d'une réaction immunologique entre ladite souche bactérienne et des anticorps dirigés contre H. pylori, présents dans l'échantillon testé.

A titre d'exemple, une détection in vitro sur un échantillon biologique pour rechercher une infection par H. pylori peut être réalisée en mettant en oeuvre les étapes suivantes :
- des plaques sont recouvertes de l'antigène utilisé pour la détection, qu'il s'agisse d'une protéine pure ou recombinante ou encore d'une souche aflagellée ou d'un extrait bactérien en particulier d'extrait NOG (N-Octyl Glucoside) de la souche N6flbA- (à titre d'exemple, la quantité d'extrait pourrait être de 3 µg/ml ou la quantité d'antigène pourrait être de 2 µg/ml),
- une gamme de témoins négatifs et positifs (le témoin positif étant mis en oeuvre avec des dilutions différentes) est utilisée et en parallèle, on teste des sérums de patients dilués au 1/100ème (volume déposé 100 µl),
- une étape d'incubation est ensuite réalisée par exemple pendant une heure à 37°C, étape suivie de plusieurs lavages successifs et d'une incubation ultérieure, par exemple pendant 1 heure à 37°C, avec un conjugué monoclonal (de type IgG humaine marquée à la peroxydase) utilisé à différentes dilutions (par exemple à une dilution de 1/32000 pour un antigène et à une dilution de 1/64000 pour un extrait bactérien), le volume déposé est de 100 µl,
- après l'incubation avec le conjugué monoclonal, différents lavages sont pratiqués (par exemple 4) et la révélation de la réaction enzymatique par « OPD + subst:at» est faite pendant 30 minutes à l'obscurité. La réaction enzymatique est ensuite arrêtée par addition de H₂SO₄ puis lecture des densités optiques DO à 492 nm/620 nm.

L'invention vise par ailleurs une composition immunogène pour l'obtention d'anticorps contre H. pylori, caractérisée en ce qu'elle comprend à titre de principe actif, une souche bactérienne selon l'invention ou un extrait de cette souche bactérienne.

Selon un mode de réalisation particulier de l'invention, une composition immunogène pour l'obtention d'anticorps contre H. pylori est caractérisée en ce qu'elle comprend une séquence d'acides aminés de la protéine FIbA.

Entre également dans le cadre de la présente invention, une composition vaccinante pour l'obtention d'anticorps protecteurs contre une infection par H. pylori, caractérisée en ce qu'elle comprend à titre de principe actif, une souche bactérienne selon l'invention ou un extrait bactérien selon les définitions précédentes.

Une autre composition vaccinante pour l'obtention d'anticorps contre une infection par H. Pylori, est caractérisée en ce qu'elle comprend à titre de principe actif, des antigènes de type uréase, en particulier des antigènes codés par les gènes ureA, ureB, ureC ou ureD et une protéine répondant à une séquence d'acides aminés définie plus haut.

L'invention vise aussi des anticorps monoclonaux ou des sérums polyclonaux dirigés contre une séquence d'acides aminés précédemment décrite. Ces anticorps sont obtenus par des techniques connues en soi et en particulier par immunisation d'un animal avec l'antigène choisi, suivie soit de la production et de la récupération des anticorps produits et de la sélection de ceux d'entre eux qui reconnaissent spécifiquement H pylori, soit de la préparation d'hybridomes par fusion de cellules spléniques de l'animal préalablement immunisé avec des cellules de myelome, ces hybridomes étant ensuite mis en culture pour obtenir des anticorps monoclonaux sélectionnés pour leur spécificité de reconnaissance par rapport à l'antigène choisi de H. pylori.

D'autres anticorps monoclonaux ou sérums polyclonaux selon l'invention sont dirigés contre une souche de H. pylori aflagellée telle que décrite dans les pages précédentes.

L'invention concerne en outre une composition pour la détection in vitro d'une infection par H. pylori dans un échantillon biologique, comprenant à titre de principe actif des anticorps monoclonaux ou un sérum polyclonal obtenus contre une souche de H. pylori de phénotype aflagellé selon l'invention.

L'invention concerne également à titre de principe actif d'un médicament, les séquences nucléotidiques codant pour les séquences d'acides aminés selon l'invention capables d'induire une réponse immunogène chez un animal ou chez un patient. Une technique pour l'utilisation de séquences nucléotidiques comme médicaments a été décrite par DONNELY et al 1995, Nature Medic. 1(6), p. 583-587.
figure 1
1A : Carte de restriction du plasmide pILL570 et du mini transposon Tn3 contenant la cassette du gène de résistance à la kanamycine.
1B : Cartes de restriction linéaires des plasmides recombinants pSUS39 et pSUS207. Les chiffres indiqués correspondent à la taille des fragments de restriction exprimée en paires de bases. H : HindIII ; Bg : BgIII. La présence d'un astérisque indique que le site de restriction a été modifié au cours du clonage et qu'il n'est plus reconnu par l'enzyme de restriction correspondant.

figure 2: Séquence nucléotidique du gène fIbA de H. pylori et séquence en acides aminés déduite, donnée en code à une lettre.

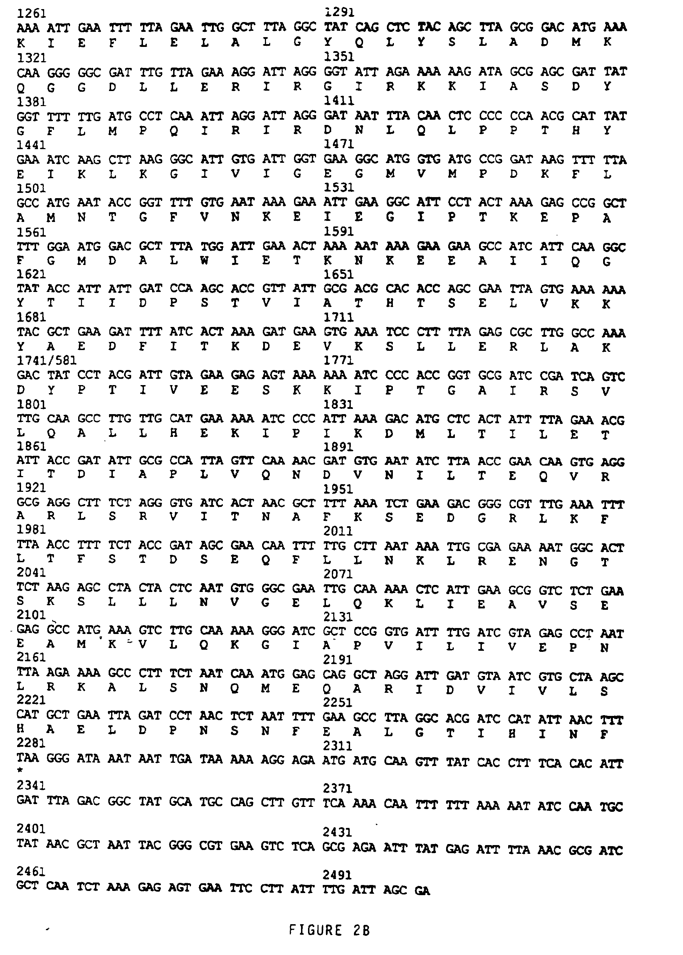

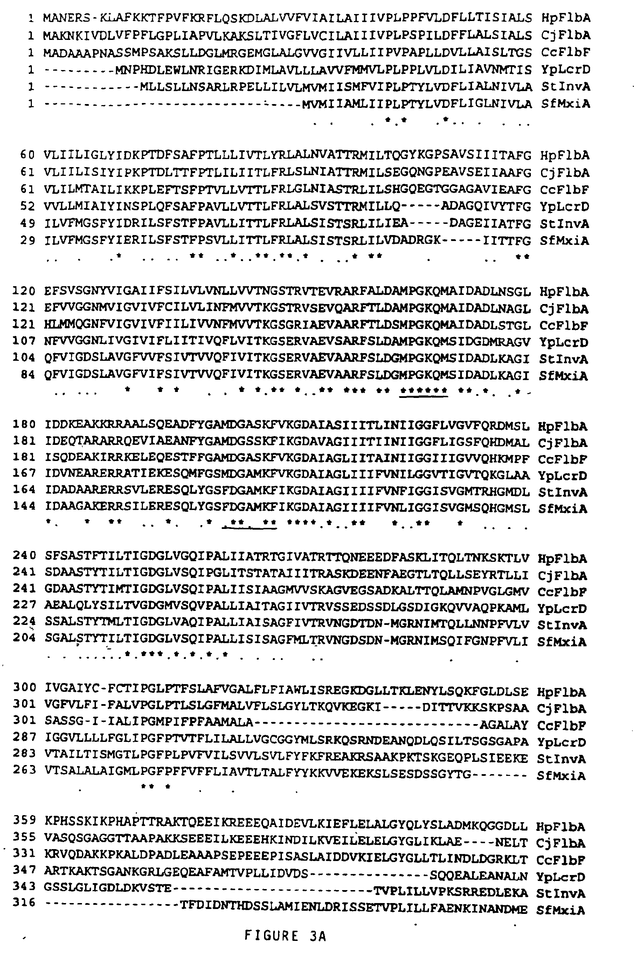

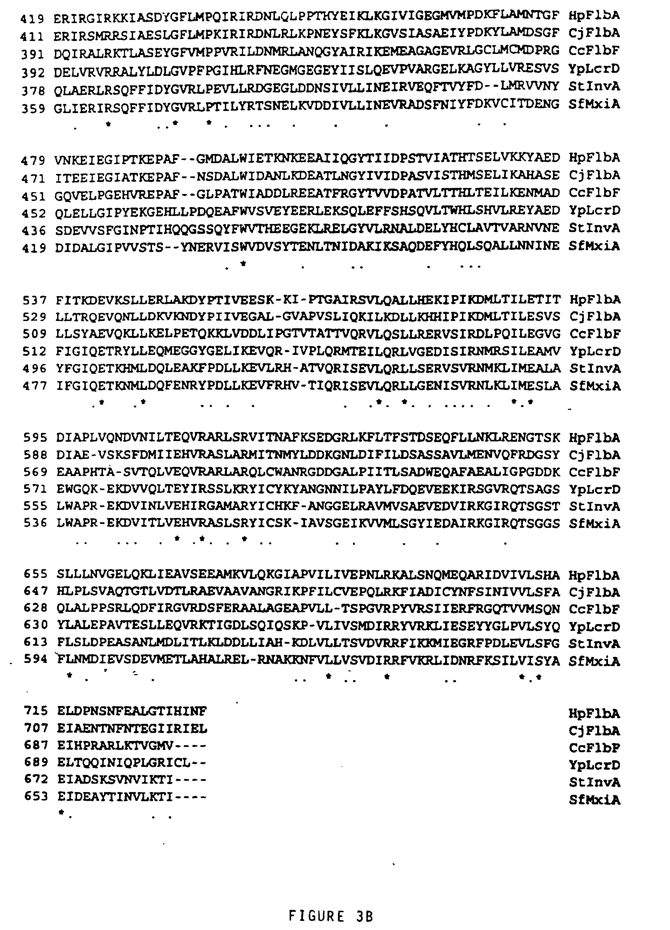

figure 3 : Alignement multiple de la protéine FIbA de H.pylori avec cinq autres membres de la famille LcrD/FIbF. CjFIbA : Campylobacter jejuni FIbA ; CcFIbF : Caulobacter crescentus FIbF ; YpLcrD : Yersinia pestis LcrD ; StInvA : Salmonella typhimurium InvA ; SfMxiA : Shigella flexneri MxiA. Les astérisques indiquent la position d'acides aminés conservés dans tous les homologues de la famille LcrD/FIbF ; les points indiquent la position d'acides aminés conservés dans au moins 5 des 6 protéines homologues ; les séquences en acides aminés conservés utilisées pour la synthèse des oligonucléotides dégénérés (OLFIbA-1 et OLFIbA-2) sont soulignées. On remarquera en particulier le degré de conservation du domaine N-terminal de ces protéines homologues qui contraste avec le degré de variabilité du domaine hydrophile de la région C-terminale.
figure 4 : Représentation schématique de l'arbre phylogénique de six protéines appartenant à la famille LcrD/FlbF. Les protéines impliquées dans la régulation de l'expression de la mobilité, FlbA de H. pylori (HpFlbA), et de Campylobacter jejuni (Cj FlbA), et FlbF de Caulobacter crescentus (Cc FlbF) forment une branche distincte de celle des protéines impliquées dans la sécrétion de protéines de virulence (InvA, MxiA et LcrD de Salmonella, Shigella et Yersinia, respectivement). Les nombres indiqués représentent la distance évolutionnaire relative.
figure 5 : Représentation schématique de la stratégie suivie pour construire les mutants isogéniques de H. pylori souche N6, mutants dans lesquels le gène codant pour la protéine FlbA a été inactivé par insertion d'un gène codant pour la résistance à la kanamycine.
figure 6 : Analyse par immunobuvardage (Western blot) des protéines d'un mutant N6-flbA⁻ à l'aide de l'antisérum AK179 (3) dirigé spécifiquement contre les flagelles purifiés de H pylori : 1 : mutant N6-flbA : 2 : double mutant flaA/flaB ; 3 : mutant flaB (8) ; 4 : mutant flaA (8) ; 5 : souche sauvage N6,

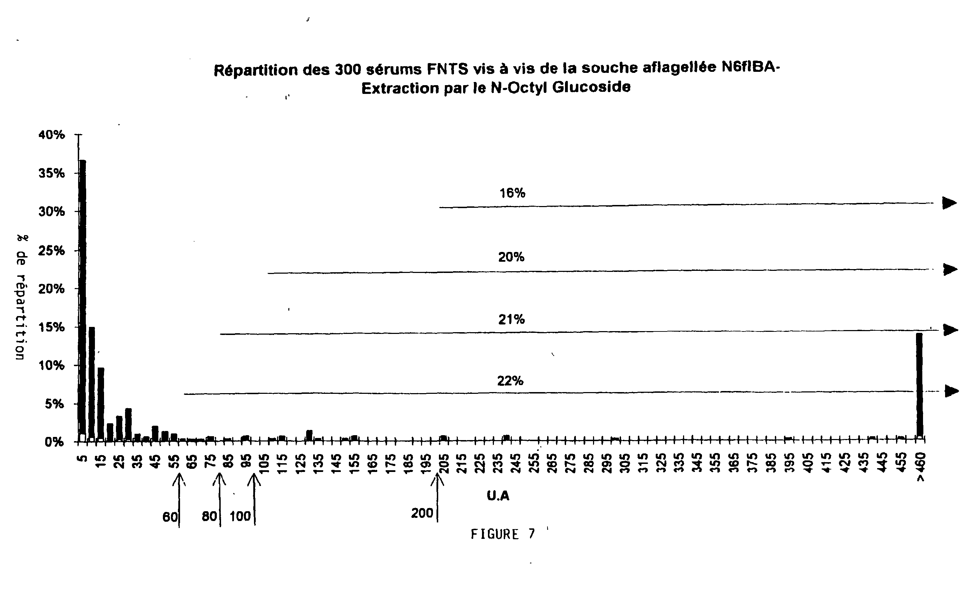

figures 7 à 11 : Résultats comparatifs de la sérologie réalisée sur H. pylori.
figures 12 et 13 : Extractions à partir de la souche aflagellée N6flbA- : les extractions ont été réalisées avec de la glycine, du PBS, ou du NOG.

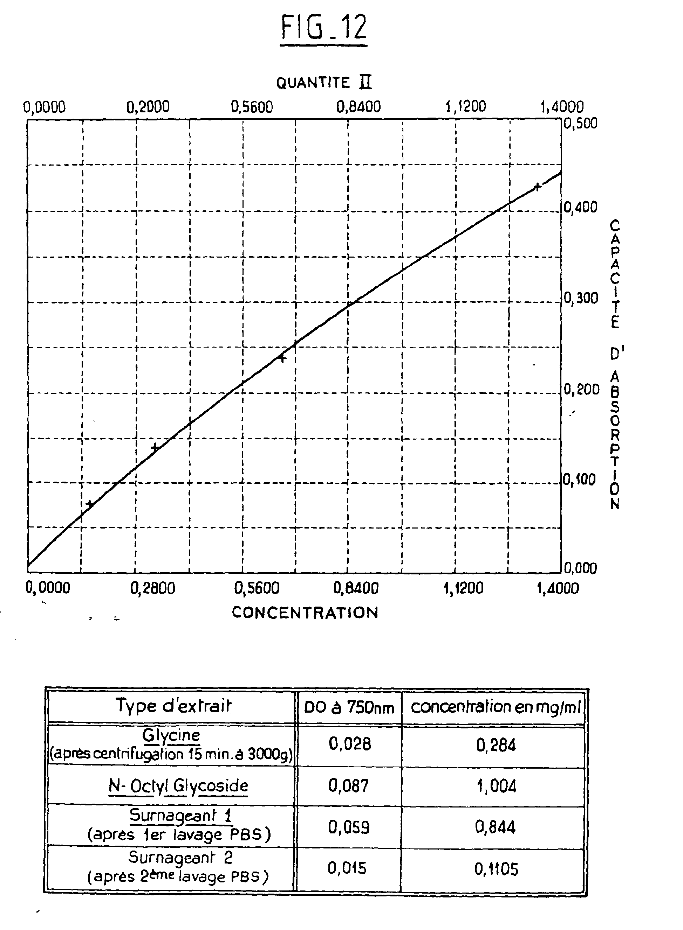

Figure 12 : Les courbes ont été réalisées à partir des données suivantes :

| | | NET ABS | CALC | | COEFFS: |
|---|---|---|---|---|---|
| STD# | CONC | 750.0 | CONC | DIFF | P2=2.0324 P1=2.2753 PO=-0 |
| 1 | 0.0000 | 0.0020 | -0.008 | 0.0080 | |
| 2 | 0.1660 | 0.0760 | 0.1721 | -0.006 | |
| 3 | 0.3300 | 0.1400 | 0.3459 | -0.016 | |
| 4 | 0.6650 | 0.2390 | 0.6474 | 0.0176 | |
| 5 | 1.3300 | 0.4280 | 1.3336 | -0.004 | |
| | | | | | MOYENNE: -1.0356E-07 |
| | | | | | S.D.: 0.0130 |

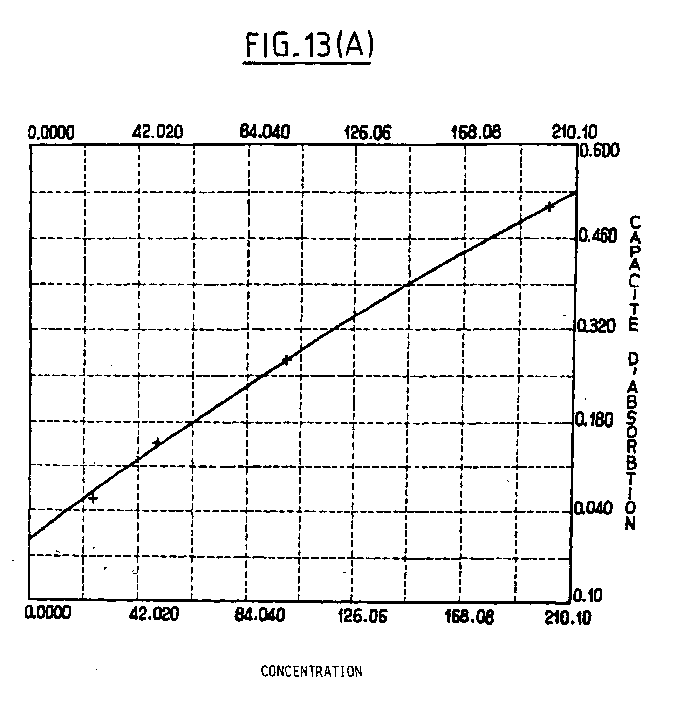

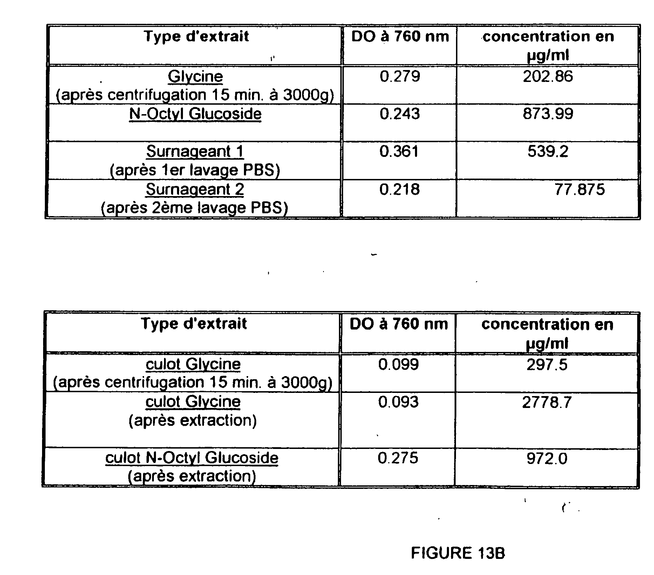

Figure 13 : Dosages protéiques en minométhode (BIOD RAD)
Glycine : dilution au 1/2 ; glucoside : dilution au 1/10 ; surnageant 1 :
dilution au 1/4 ; surnageant 2 : non dilué.

Les courbes ont été réalisées à partir des données suivantes :

| | | NET ABS | CALC | | COEFFS: |
|---|---|---|---|---|---|
| STD# | CONC | 750.0 | CONC | DIFF | P2=144.63 P1=314.31 PO=2.4815 |
| 1 | 0.0000 | -0.003 | 1.5398 | -1.540 | |
| 2 | 25.000 | 0.0600 | 21.861 | 3.1392 | |
| 3 | 50.000 | 0.1470 | 51.810 | -1.810 | |
| 4 | 100.00 | 0.2750 | 99.855 | 0.1454 | |
| 5 | 200.00 | 0.5090 | 199.94 | 0.0636 | |

### EXEMPLES

### 1 Identification du gène fIbA et préparation de souches aflagellées

Parmi les protéines connues pour jouer un rôle dans la régulation de l'expression de la mobilité chez les bactéries, les protéines appartenant à la famille LcrD/FIbF récemment identifiée, et qui inclut la protéine LcrD des bactéries du genre Yersinia (6), la protéine InvA de Salmonella (2), MxiA de Shigella (1), FIbF de Caulobacter crescentus (7) et FIbA de Campylobacter jejuni (4) sont des protéines intéressantes. Les protéines LcrD, InvA et Mxia sont impliquées dans la régulation et/ou la sécrétion de protéines associées à la virulence des bactéries qui les expriment, alors que les protéines FlbF de Caulobacter crescentus et FIBA de Campylobacter ieiuni sont impliquées dans la régulation de la biosynthèse des flagelles et donc de la mobilité. Les homologues connus à ce jour de la famille LcrD/FlbF ont des domaines très conservés surtout dans la partie N-terminale de ces protéines, et deux de ces régions conservées (MPGKQM, acides aminés 151 à 156 de la protéine LcrD de Yersinia) et MDGAMKF (acides aminés 189 à 195 de LcrD) ont ainsi pu être utilisées pour la définition de deux oligonucléotides dégénérés (OLFIbA-1 et OLFlbA-2, tableau 1) qui ont été synthétisés et ont servi d'amorces nucléotidiques dans des expériences d'amplification génique réalisées à partir de l'ADN chromosomique d'Helicobacter pylori. Un fragment de 130 paires de base (pb) a ainsi pu être amplifié et la détermination de sa séquence nucléotidique a montré que ce fragment codait pour un segment d'une protéine très homologue aux protéines de la famille LcrD/FlbF. Ce fragment amplifié a alors été marqué radioactivement et utilisé comme sonde pour cribler une banque cosmidique de H.pylori.

Ce fragment correspond à la séquence comprise entre les nucléotides 575 et 707 de la séquence représentée à la figure 2.

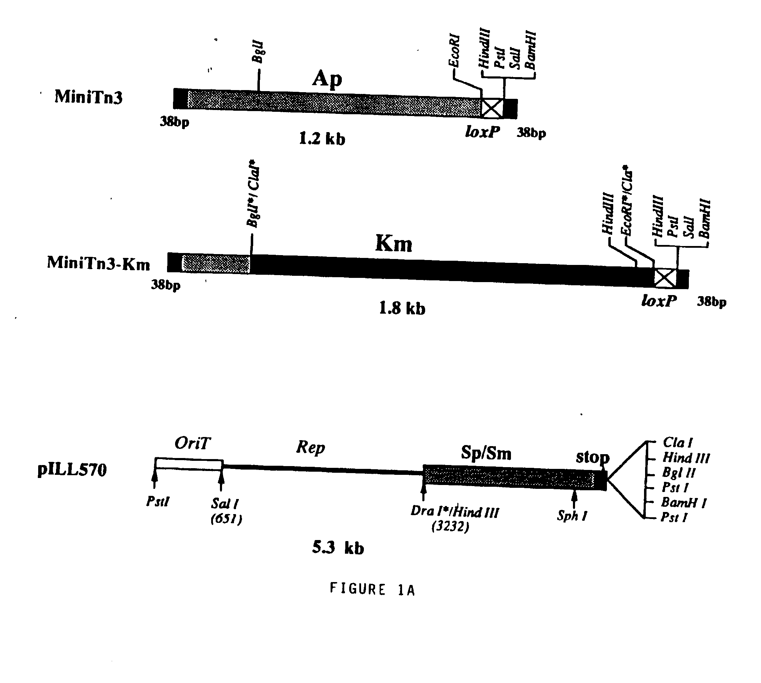

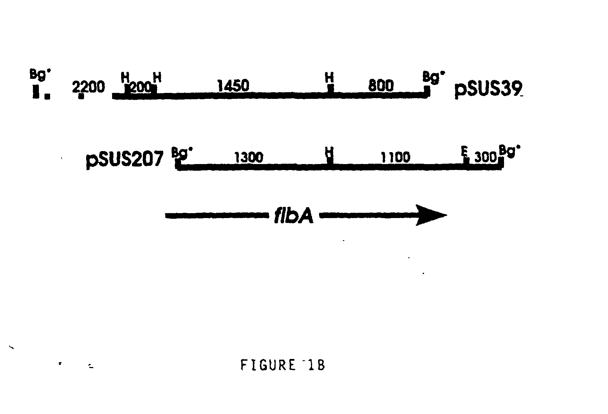

Un des cosmides de la banque génomique a été identifié comme codant pour l'homologue LcrD/FlbF de H. pylori, et a ensuite été soumis à une digestion partielle par Sau3A de façon a réaliser une mini banque (200 sous-clones) du cosmide dans le vecteur plLL570 contenant des fragments insérés ayant une taille comprise entre 2 et 5 (kilobases). Le vecteur plLL570 a été décrit dans la publication de Labigne A. et al (Institut Pasteur/Elsevier Paris 1992. Res. Microbiol. 1992, 143, 15-26). Sa carte de restriction est donnée à la figure 1A. Ces 200 clones ont alors été hybridés à la sonde de 130 pb et les clones hébergeant les plasmides pSUS39 et PSUS207 ont donné une hybridation positive. La carte de restriction linéaire de ces deux plasmides recombinants est représentée dans la figure 1B et montre que les deux inserts de ces clones ont des séquences chevauchantes. La détermination de la séquence nucléotidique de ces deux inserts a révélé qu'aucun des deux ne contenait le gène fIbA dans son entièreté. Le gène fIbA de H. pylori ainsi désigné du fait de son homologie avec le gène fIbA de Campylobacter jejuni correspond à une phase ouverte de lecture de 2196 nucléotides et code pour une protéine de masse moléculaire calculée de 80,1 kilodaltons. La séquence nucléotidique de flbA et la séquence en acide aminés de FlbA sont données dans la figure 2. Il n'a pas été détecté en amont et en aval de la phase ouverte de lecture de séquences de consensus caractéristique de séquences promoteurs ou terminateurs.

La protéine FIbA présente des similitudes avec les protéines FIBA de Campylobacter jejuni et FIbF de Caulobacter crescentus, toutes deux impliquées dans la mobilité (51,7 % et 40,4 % d'identité, respectivement) alors que ces pourcentages sont moins élevés avec les membres de la famille protéique LcrD/FIbF qui ne sont pas impliqués dans la mobilité : 32,8 % d'identité avec LcrD de Yersinia, 30,5 % avec MxiA de Shigella et 29,3 % avec InvA de Salmonella. Un alignement multiple de la séquence en acides aminés de ces protéines avec celle de FlbA de H. pylori est donné dans la figure 3. Les régions les plus conservées des homologues de la famille LcrD/FIbF se trouvent dans la partie N-terminale des protéines.

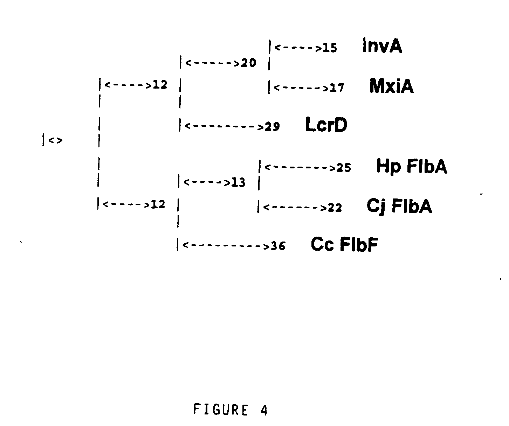

L'évolution phylogénétique des protéines impliquées dans la mobilité (FlbA et FlbF) et celle des protéines impliquées dans la régulation de l'expression et/ou la sécrétion de protéines associées à la virulence est représentée schématiquement par un arbre phylogénique (figure 4). Deux branches distinctes sont visibles ; FlbA de H. pylori appartenant sans ambiguité à la branche correspondant aux protéines de régulation impliquées dans la biosynthèse des flagelles.

### Construction et caractérisation de mutants isogénigues de H pylori déficients dans la synthèse de la protéine FlbA.

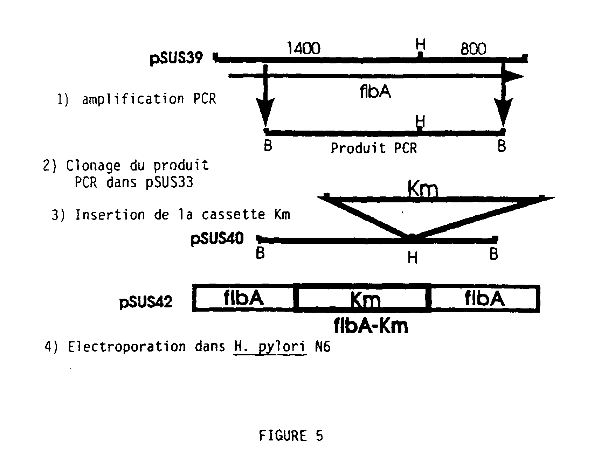

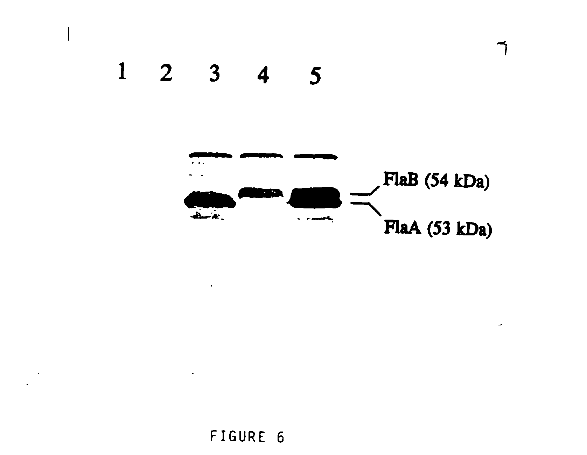

Un fragment de 1600 paires de bases a été amplifié à partir du plasmide pSUS39 à l'aide des oligonucléotides OLFlbA-7 et OLFlbA-8 (Tableau 1), contenant chacun à leur extrémité 5' des sites de restriction BamHl. Ce fragment amplifié contient dans sa région centrale un site unique de restriction par l'endonucléase Hindlll et a été cloné dans le vecteur pSUS33, un dérivé du plasmide pUC19 dans lequel le site situé dans le site de polyclonage Hindlll a été délété. Pour obtenir pSUS33, le plasmide pUC19 a été restreint par Hindlll les extrémités collantes résultant de cette restriction ont été traitées avec l'enzyme de Klenow et l'ADN T4 polymérase de façon à produire des extrémités franches ; le fragment résultant a été religaturé avec l'ADN T4 ligase et introduit dans E. coli DH5x pour produire pSUS33. Le plasmide recombinant résultant de l'intégration du fragment de 1600 paires de base dans pSUS33 a été désigné pSUS42 ; il a été linéarisé par Hindlll, ses extrémités ont été transformées en extrémité à bouts francs et la cassette smal - kanamycine provenant du plasmide pILL600 (Labigne A. et al, 1988, J. Bact., 170, 1704-1708) a été clonée dans ce site unique donnant naissance au plasmide pSUS42. Le plasmide pSUS42 a ensuite été introduit par électroporation dans la souche "N6" de H. pylori. L'électroporation a été réalisée en suivant la technique décrite par Ferrero R. L. et ai (Journal of Bacteriology, July 1992, p. 4212-4217, vol. 174, n° 13). Les transformants obtenus après sélection sur un milieu sélectif contenant de la kanamycine (25 µg/ml) ont ensuite été caractérisés génotypiquement et phénotypiquement. La figure 5 montre un schéma de la procédure suivie pour réaliser la construction de mutants. La caractérisation génotypique de ces mutants par amplification génique et hybridation de type Southern a montré que le génome des transformants résistants à la kanamycine contenait le gène de résistance inséré au milieu du gène flbA, et donc qu'il y avait eu remplacement allélique par double crossing-over de la copie sauvage du gène flbA par la copie inactivée flbA-Km avec perte des séquences nucléotidiques du vecteur pSUS33. La caractérisation phénotypique des mutants flbA⁻ de H. pylori a montré qu'ils étaient non mobiles ; par ailleurs, leur analyse par microscopie électronique révélait qu'il y avait absence totale des éléments du flagelle, et absence de la gaine du flagelle. Les expériences d'immunobuvardages (Western blots) réalisées avec des anticorps dirigés contre les protéines des flagelles totales de H. pylori (Figure 6) montraient l'absence des deux bandes peptidiques correspondant aux sous-unités flagellaires FIaA et FIaB, ainsi qu'une bande correspondant à un polypeptide d'une masse apparente de 90 kilodaltons, protéine récemment identifiée comme la protéine du crochet (ou protéine d'ancrage) du flagelle par O'Toole et collaborateurs (5).

L'ensemble de ces résultats suggère que la protéine FIbA de H. pylori est essentielle pour la biosynthèse de toutes les structures flagellaires, et que l'activation du gène codant pour cette protéine, résulte en l'arrêt complet de la synthèse de toute structure entrant dans la constitution du flagelle et non pas dans l'interruption de l'exportation des constituants de ces structures.

**Tableau 1 : Oligonucléotides utilisés dans cette étude**

| Oligonucléotides | Position | Brin | Séquence nucléotidique |
|---|---|---|---|
| OLFlbA-1 | AS 151-156(LcrD) | + | ATGCCTCGAGGTCGAAAAGCAAGATG |
| OLFlbA-2 | AS 189-195 (LcrD) | - | GAAATCTTCATACTGGCAGCTCCAGTC |
| OLFlbA-7 | 515-534 | + | CGGGATCCGTGGTTACTAATGGTTCTAC |
| OLFlbA-8 | 2092-2111 | - | CGGGATCCTCATGGCCTCTTCAGAGACC |

II Sérologie H. pylori

### 7 - modèles étudiés

1) HspAmalE protéine recombinante de 47,5 kD (HspA=13 kD) Sur la population dite population 1 de sérums documentés, une sensibilité de 41% et une spécificité de 96% avaient été obtenues.
2) N6fIbA- souche aflagellée d'Helicobacter pylori 3 extractions ont été réalisées : -N-Octyl Glucoside
   -PBS
   -Glycine
   L'extraction au N-Octyl Glucoside (NOG) semble pour l'instant la meilleure.
3) -N6 souche sauvage correspondante Une extraction au N-Octyl Glucoside a été réalisée.

Une deuxième population de sérums a été mise en oeuvre (population II). Il s'agit d'une centaine de sérums bien documentés du point de vue clinique, endoscopique, histologique, bactériologique et anatomopathologique. C'est grâce à cette population Il que les performances des différents modèles étudiés ont été appréciées. Cinq populations différentes ont été testées.

### - 5 populations de sérums testées :

- 300 sérums tout venant (FNTS)
- 18 sérums positifs par la sérologie WHITTAKER (CBMS)
- 92 sérums bien documentés dits sérums de la population II
- 87 sérums documentés du point de vue bactériologique et anatomopathologique dits sérums de la population I.
- 23 sérums pouvant présenter des réactions croisées :
   - 10 sérums anti-Legionella positifs
   - 10 sérums anti-Chlamydia positifs
   - 3 sérums anti-Campylobacter positifs

En parallèle, deux kits concurrents ont été testés, qui d'après les études bibliographiques sont performants.
- 2 kits commerciaux testés :
   - Cobas Core (ROCHE)
   - Pylori Stat (WHITTAKER)
- Résultats

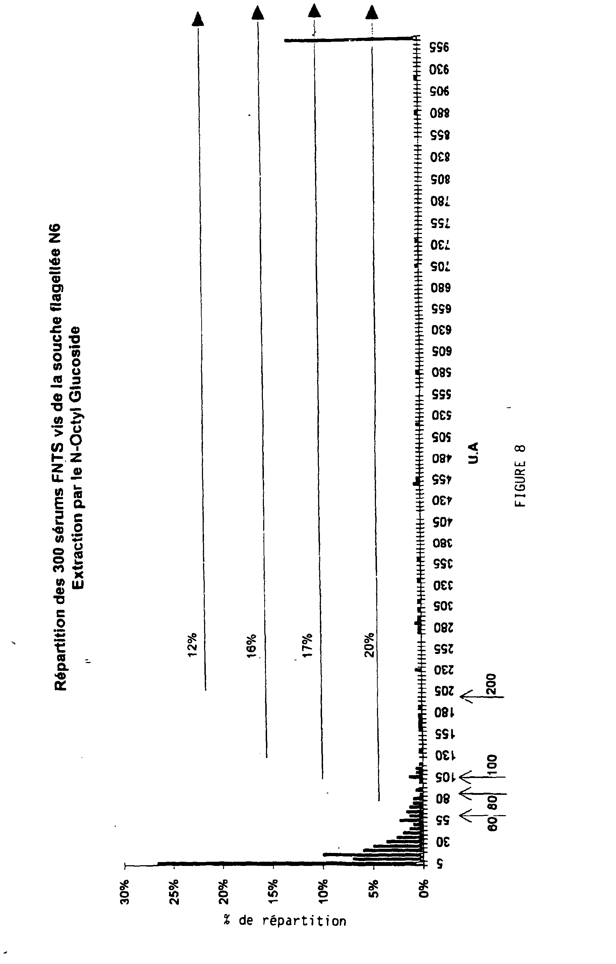

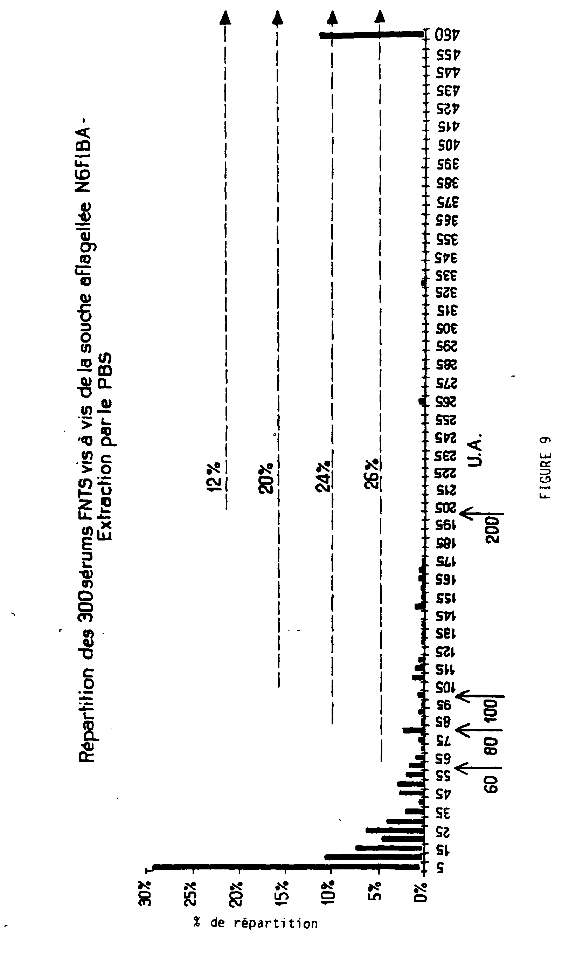

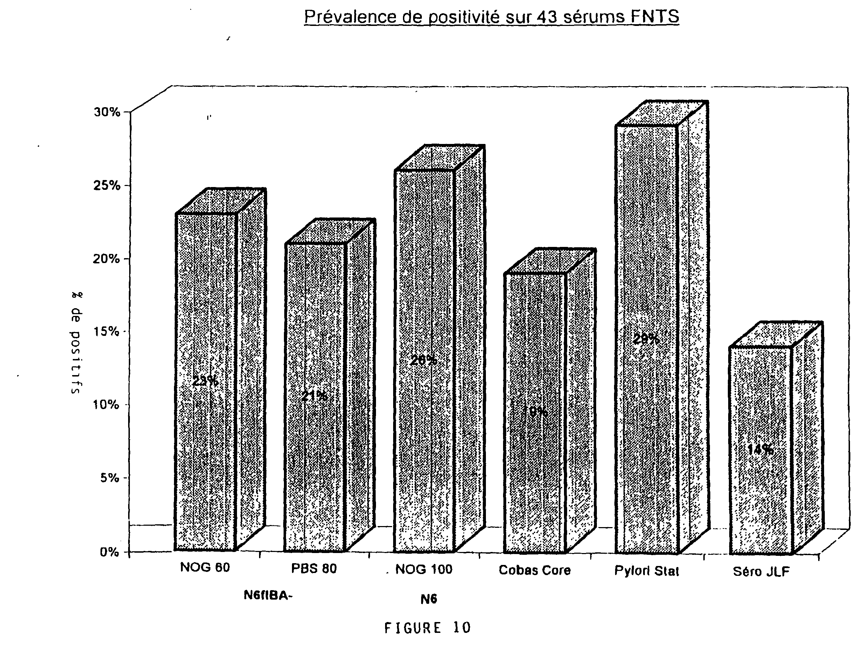

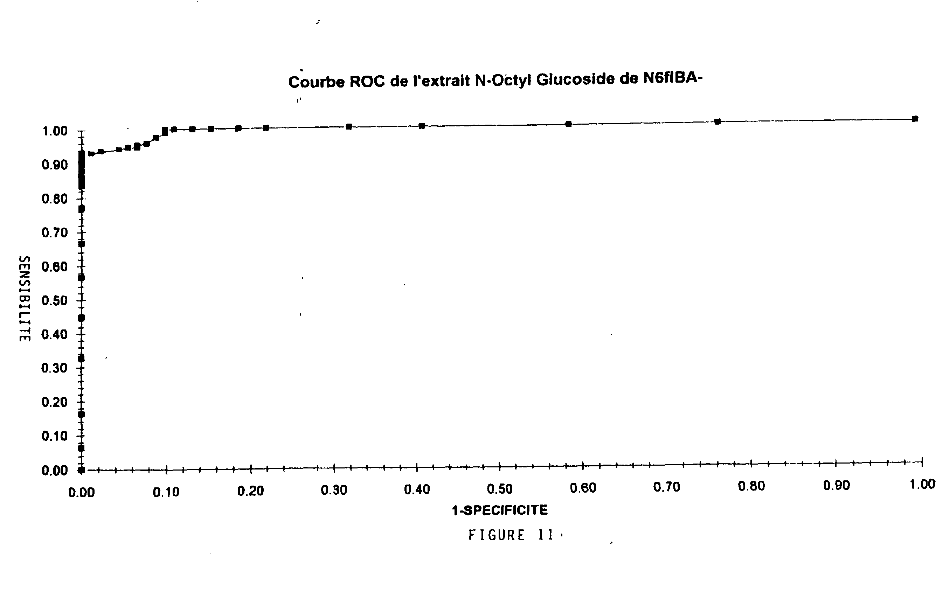

Les sérums tout venant (FNTS) (Figures 8 à 11, tableau 2)
- 300 sérums ont été passés dans les modèles suivants :
   - Hsp A malE
   - N6 fIBA-
   - N6

Les études épidémiologiques donnent des séroprévalences, en France, entre 20 et 25%. La répartition de 300 sérums de donneurs de sang a été étudiée et on a calculé la prévalence de positivité pour différentes valeurs seuil, afin de valider la valeur seuil préalablement définie sur la sérothèque du CBMS (sérologie WHITTAKER).

Cette étude permet de comparer les différents tests avec une même séroprévalence.
- Les 43 premiers sérums ont également été passés dans les modèles suivants :
   - Cobas Core (ROCHE)
   - Pylori Stat (WHITTAKER)
   - sérologie dite JLF (test ELISA, basé sur un extrait
   aqueux de plusieurs souches bactériennes)

Les résultats sont exprimés en unité arbitraire, et pour différentes valeurs seuil, un résultat positif est noté 1, un résultat négatif est noté 0.

En comparant ces 43 sérums vis à vis des différents tests, on peut constater que :
- la souche aflagellée N6flbA- et le test Cobas Core (Roche) ont des séroprévalences comparables de l'ordre de 20%.
- HspA a une séroprévalence très faible (7%) ce qui laisse supposer un manque de sensibilité, compte tenu des résultats ultérieurs.
- la sérologie JLF parait très spécifique puisque la séroprévalence n'est que de 14%, en tenant compte des résultats ultérieurs.
- le test Pylori Stat (Whittaker) donne une séroprévalence élevée (29%) qui montrerait un manque de spécificité, ou une valeur seuil trop basse.

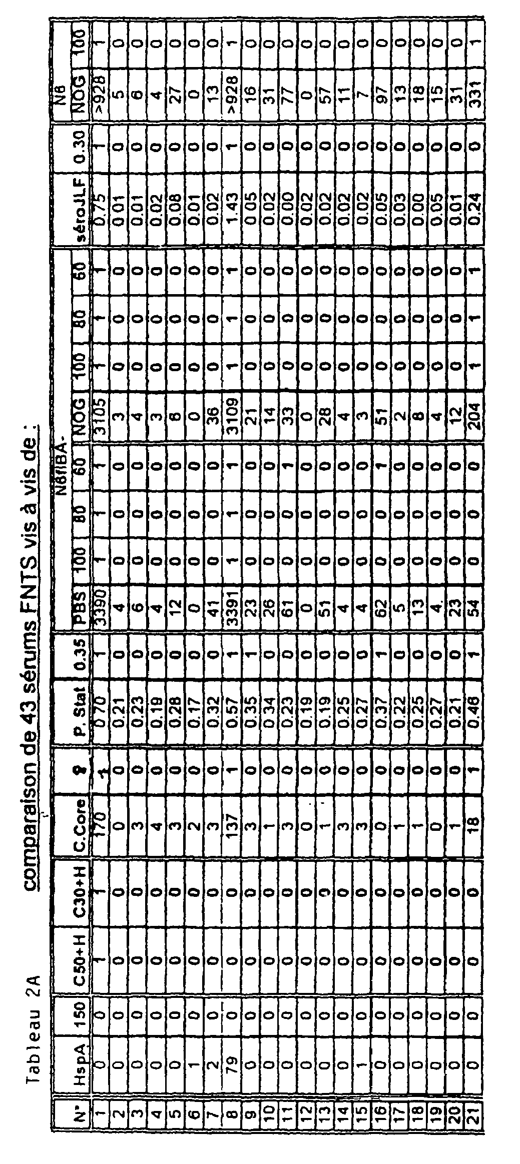

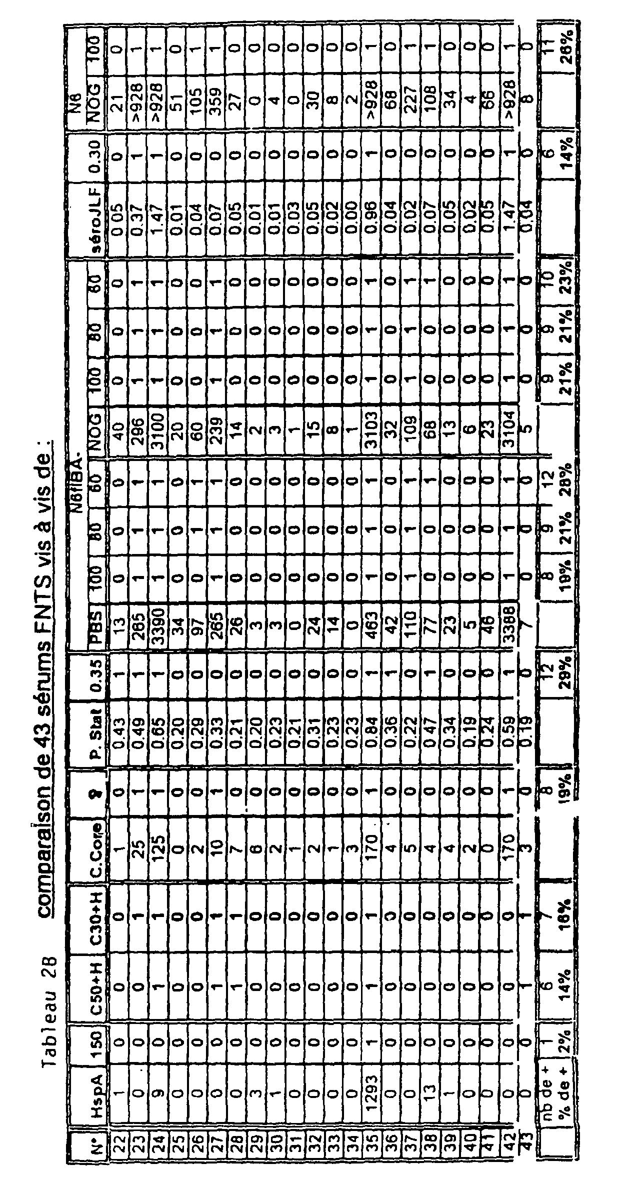

### Les sérums positifs en sérologie WHITTAKER (CBMS) (tableau 3)

Trois sérums se sont révélés positifs uniquement avec le test Pylori Stat (Whittaker). Ils n'ont été confirmés par aucun autre test.
On peut supposer qu'il s'agit là d'un manque de spécificité de ce test.
Si l'on prend comme référence le test Cobas Core (Roche) qui est l'un des meilleurs actuellement sur le marché, nous pouvons comparer nos différents modèles par rapport à Cobas Core.
- La souche aflagellée N6fIbA- est parfaitement corrélée à Cobas Core.
- Les 3 sérums négatifs Cobas Core le sont également avec N6flbA-
- Les 15 sérums positifs Cobas Core le sont aussi avec N6flbA-
- La souche sauvage N6 montre les mêmes performances que la souche aflagellée.
- HspA manque aussi de sensibilité car 9 sérums positifs Cobas
   Core sont négatifs avec HspA.

Les 3 sérums négatifs Cobas Core le sont aussi avec HspA.

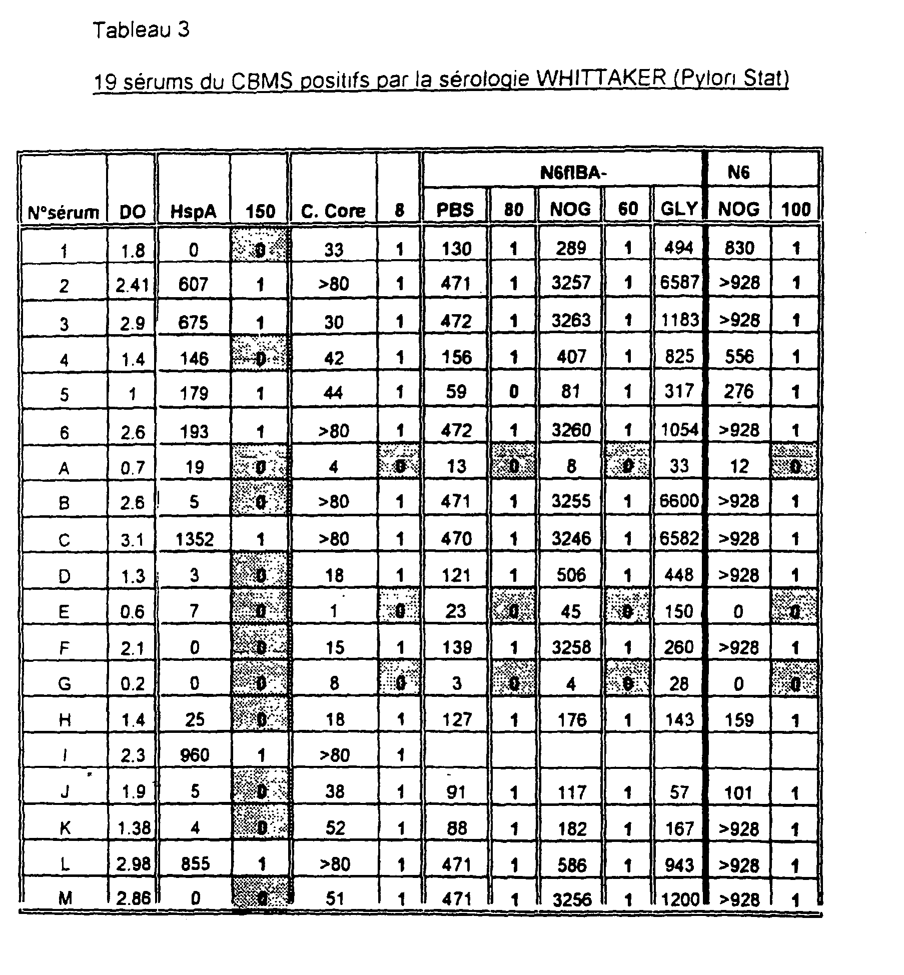

Les sérums de la population II
92 sérums ont été sélectionnés et se répartissent en 3 groupes :
- 34: patients dyspeptiques
   diagnostic d'ulcère (duodénal ou gastrique) à l'endoscopie et à l'histologie
   présence d'Helicobacter pylori par culture et/ou anapath ; un test rapide à l'urée a également été réalisé.
   Ce groupe sera noté Hp+/U+
- 27 : patients dyspeptiques
   diagnostic différent d'ulcère (gastrite...) à l'endoscopie et à l'histologie
   présence d'Helicobacter pylori par culture et/ou anapath ; un test rapide à l'urée a également été réalisé.
   Ce groupe sera noté Hp+/U-
- 31 : patients dyspeptiques ou non
   gastro-duodénum normal à l'endoscopie et à l'histologie
   absence d'Helicobacter pylori par culture et anapath ; un test rapide à l'urée a également été réalisé.
   Ce groupe sera noté Hp-

Pour chaque patient, les données cliniques, endoscopiques, histologiques, bactériologiques et anatomopathologiques sont indiquées.
Cette population bien documentée a permis de définir des critères de
Sensibilité et de Spécificité.
- HpA : On note toujours un manque de sensibilité important comme constaté avec la population I.
La sensibilité est de 59% pour une spécificité de 100.
- N6fIbA- : On vérifie une sensibilité de 100% pour l'extrait au N-Octyl Glucoside avec une spécificité de 90%.
La performance est comparable au test Cobas Core de Roche (sensibilité de 98% pour une spécificité de 94%).
- N6 : La souche sauvage est tout à fait comparable à la souche aflagellée sur la population II.
Aucun des 31 sérums négatifs n'est positif avec la souche sauvage ; aucune réaction croisée due au flagelle n'a été détectée sur cette population II.

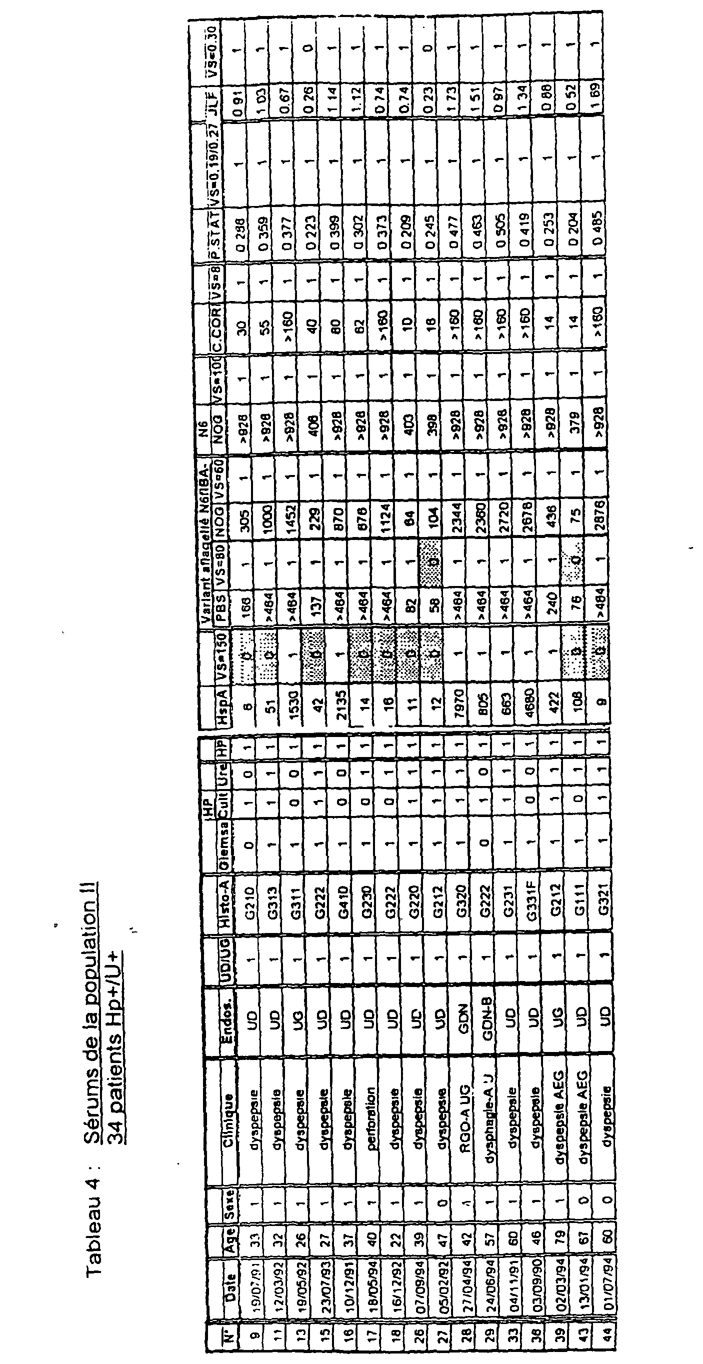

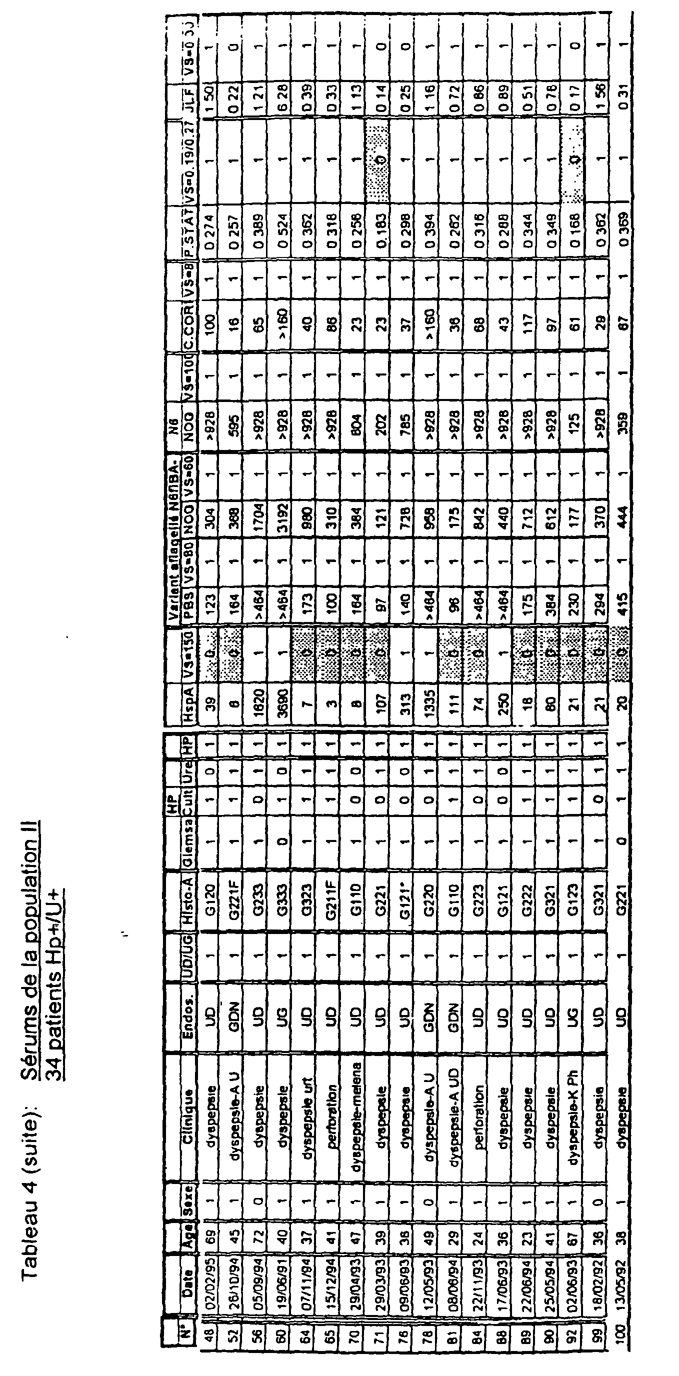

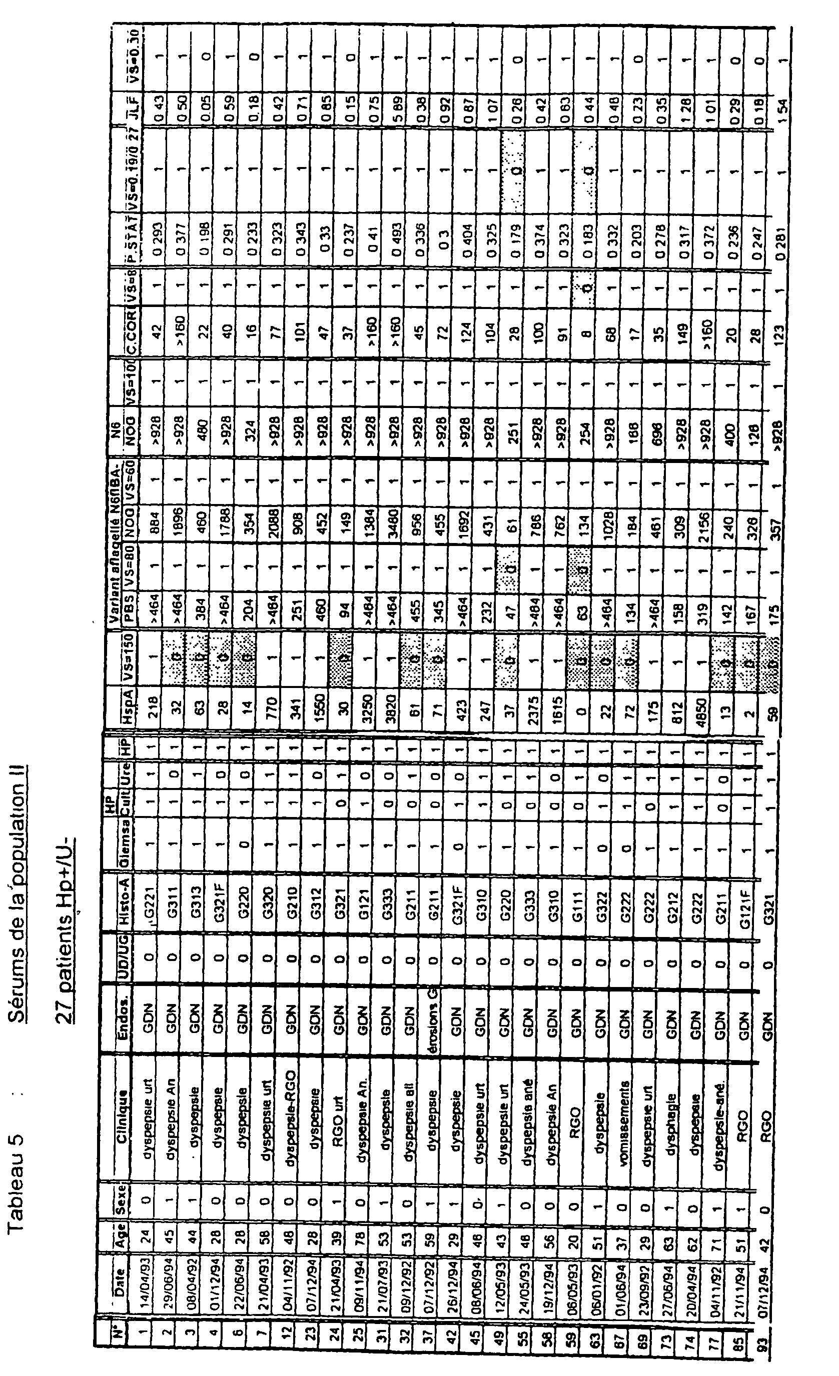

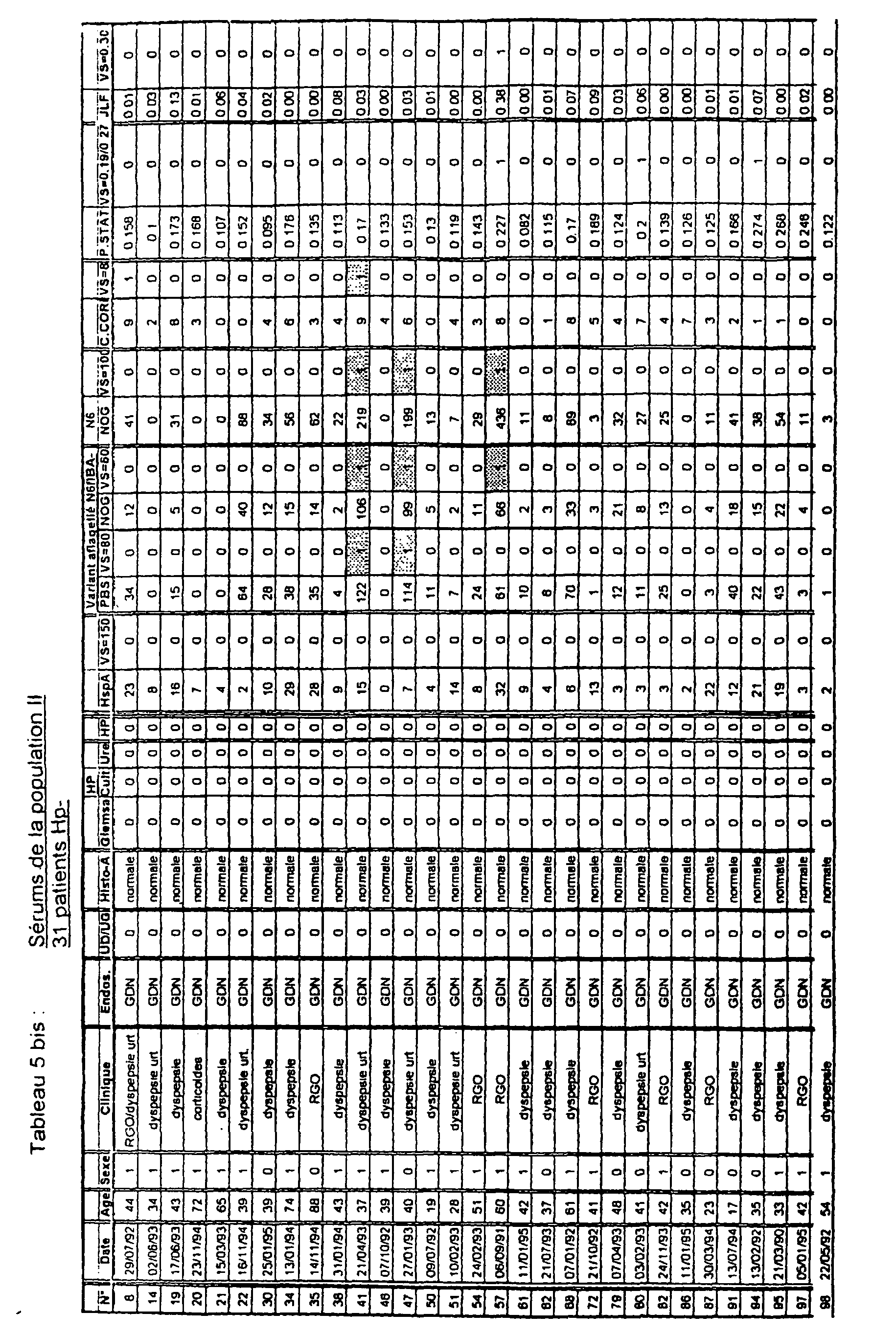

**Tableau 6 Sérums de population II Par rapport à la présence de Hp (culture et/ou anapath) et Ulcère**

| | | | | Sensibilité | Spécificité |
|---|---|---|---|---|---|
| Par rapport à Hp+ et UD/UG soit 34Hp+/U+ | | HspA malE | VS=100 | 44 1% (15/34) | *100% (31*/*31)* |
| | | | VS=50 | 52.9% (18/34) | *100% (31*/*31)* |
| | | | VS=20 | 64.7% (22/34) | *73.8% (25*/*31)* |
| | N6flbA- | NOG | VS=100 | *94.1%* (32/34) | *96.8% (30*/*31)* |
| | | | VS=80 | 94.1% (32/34) | *93.6% (29*/*31)* |
| | | | VS=60 | 100% (34/34) | *90.3% (28*/*31)* |
| | | PBS | VS=100 | 82.4% (28/34) | *93.6% (29*/*31)* |
| | | | VS=80 | 94.1% (32/34) | *93.6% (29*/*31)* |
| | | | VS=60 | 97.1% (33/34) | *83.9%* (26/31) |
| | | Séro JLF | VS=0.30 | 82.4%(28/34) | *96.8%* (30/31) |
| | | Pylori Stat | | 94.1 % (32/34) | *90.3 % (28*/*31)* |
| | | Cobas Core | | 100% (34/34) | *93.6%* (29/31) |

**Tableau 7 : Sérums de population II Par rapport à la présence de Hp (culture et/ou anapath)**

| | | | | **Sensibilité** | **Spécificité** |
|---|---|---|---|---|---|
| Par rapport à Hp+ - 34 UD/UG - 27 GNU soit : 61 Hp+ 31 Hp- | | HspA malE | VS=100 | 45.9% (28/61) | *100% (31*/*31)* |
| | | | VS=50 | 59% (36/61) | *100% (31*/*31)* |
| | | | VS=20 | 80.7% (45/61) | 73.8% *(25*/*31)* |
| | N6flbA- | NOG | VS=100 | 95.1% (58/61) | *96.8% (30*/*31)* |
| | | | VS=80 | 95.1 % (58/61) | *93.6% (29*/*31)* |
| | | | VS=60 | 100% (61/61) | 90.3% *(28*/*31)* |
| | | PBS | VS=100 | 85.3% (52/61) | *93.6% (29*/*31)* |
| | | | VS=80 | 93.4% (57/61) | *93.6% (29*/*31)* |
| | | | VS=60 | 96.7% (59/61) | *83.9% (26*/*31)* |
| | | Séro JLF | VS=0.30 | 78.7%(48/61) | *96.8% (30*/*31)* |
| | | Pylori Stat | | 93.4% (57/61) | *90.3% (28*/*31)* |
| | | Cobas Core | | 93.3%(60/61)* | *93.6% (29*/*31)* |

| | | | | | |
|---|---|---|---|---|---|
| ***** sérum = VS | | | | | |

**Tableau 8 : Sérums de population II Par rapport à la présence de Hp (culture et/ou anapath) et l'absence d'Ulcère**

| | | | | **Sensibilité** | **Spécificité** |
|---|---|---|---|---|---|
| Par rapport à Hp+ et GNU soit : 27 Hp+/U- | | HspA malE | VS=100 | 48.2% (13/27) | *100% (31*/*31)* |
| | | | VS=50 | 66.7% (18/27) | *100% (31*/*31)* |
| | | | VS=20 | 85.2% (23/27) | *73.8% (25*/*31)* |
| | N6flbA- | NOG | VS=100 | 96.3% (26/27) | 96.8% *(30*/*31)* |
| | | | VS=80 | 93.6% (26/27) | 93.6% *(29*/*31)* |
| | | | VS=60 | 100% (27/27) | *90.3% (28*/*31)* |
| | | PBS | VS=100 | 88.9% (24/27) | *93.6%* (29/31) |
| | | | VS=80 | 92.6% (25/27) | *93.6% (29*/*31)* |
| | | | VS=60 | 96.3% (26/27) | *83.9% (26*/*31)* |
| | | Séro JLF | VS=0.30 | 74.1%(20/27) | *96.8%* (30/31) |
| | | Pylori Stat | | 92.6% (25/27) | *90.3% (28*/*31)* |
| | | Cobas Core | | 96.3%(26/27) | *93.6% (29*/*31)* |

La place de la sérologie
La sérologie est placée à 2 niveaux :
- Sérologie très sensible : afin de dépister le portage de la bactérie chez les sujets jeunes se plaignant de douleurs épigastriques.
   Si la sérologie s'avère négative, le sujet de subira pas d'endoscopie ni de biopsie, et une autre cause à ses douleurs sera recherchée.
- Sérologie spécifique de risque : il s'agit là de mettre en évidence le risque de faire une infection grave à Helicobacter pylori c'est à dire un ulcère, un cancer ou un lymphome gastrique (lymphome du MALT).
- soit en utilisant une molécule spécifique du risque en question
- soit en utilisant un seuil spécifique de risque
   (valeur seuil plus élevée chez les sujets à risque que chez les sujets non à risque).

Cette sérologie spécifique peut être utilisée pour faire un screening de la population générale, et ainsi de dépister les cancers et les lymphomes, liés à Helicobacter pylori qui ne seraient pas dépistés par fautes de symptômes. (Seuls les sujets se plaignant de douleurs vont consulter un gastro-entérologue).

La réponse à la question de sensibilité est bonne.

**Tableau 9 : Moyenne et écart type des U.A. dans les 3 groupes de patients.**

| | | **Hp-(n=31)** | **Hp+/U- (n=27)** | **Hp+/U+ (n=34)** |
|---|---|---|---|---|
| Hsp A | moyenne | 10.61 | 775.72 | 770.32 |
| | écartype | *8.81* | *1312.56* | *1666.52* |
| N6fIBA- (NOG) | moyenne | 17.16 | 895.50 | 944.85 |
| | écartype | *26.69* | *818.57* | *915.27* |

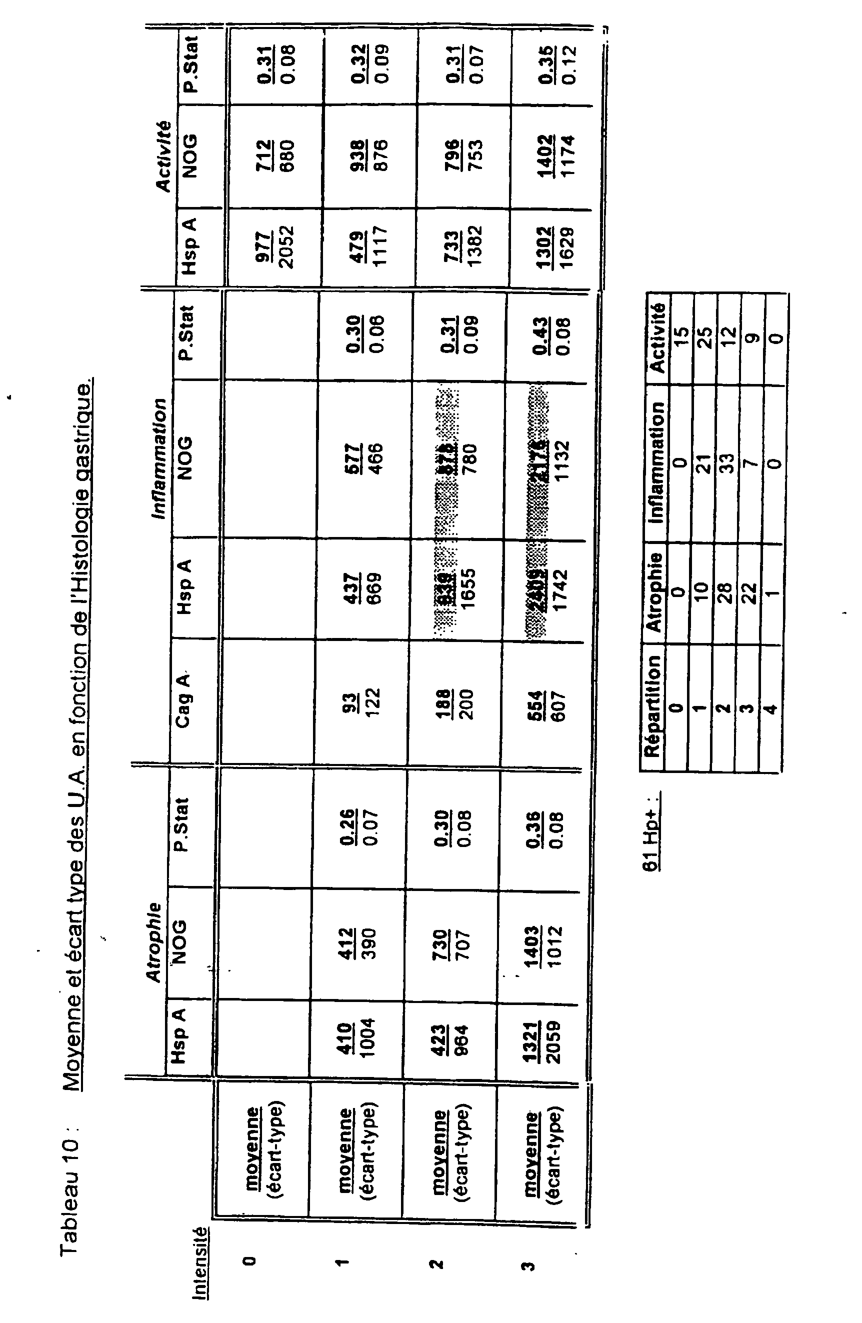

Corrélation entre l'intensité de la gastrite et le taux en anticorps
La gastrite se définit par 3 paramètres :
- L'atrophie (représentée par le 1er chiffre après G) ; son intensité est notée de 1 à 4.
- L'inflammation globale correspond à l'infiltration de Polynucléaires neutrophiles et de Monocytes ; (représentée par le 2ème chiffre après le G). Son intensité est notée de 1 à 3.
- L'activité correspond au nombre de Polynucléaires neutrophiles (représentée par le 3ème chiffre après le G) ; son intensité est notée de 0 à 3. Certaines formes folliculaires sont notées F.
Normalement, on peut observer la corrélation suivante :
L'activité est très bien corrélée à Helicobacter Pylori.
L'inflammation est bien corrélée à Helicobacter Pylori.
On a donc calculé en fonction de ces 3 paramètres et de leur intensité, la moyenne des titres observés dans chaque groupe.

### Interprétation des résultats :

L'utilisation d'un test t permettra de mettre en évidence une différence significative ou non entre 2 moyennes observées avec un risque de 5%.
L'hypothèse sur laquelle est fondée le test t est l'égalité des variances, mise en évidence par un test F (test de Fisher).
Certaines variances n'étant pas égales,- on ne pourra donc pas comparer toutes les moyennes entre elles.
Les comparaisons des moyennes, lorsque cela est possible, ont pu mettre en évidence des différences significatives ou non dans les différents groupes.
- Différence significative :
Dans le groupe « Inflammation », entre les moyennes des « 2 » et « 3 » de HspA et de NOG.
- Différence non significative :
Au niveau de l'activité, on n'a pas mis en évidence de différence significative entre les différents niveaux d'intensité :

| | |
|---|---|
| - HspA : pas de différence significative entre les niveaux | 0 et 2 |
| | 0 et 3 |
| | 1 et 2 |
| | 1 et 3 |
| | 2 et 3 |
| - NOG : pas de différence significative entre les niveaux | 0 et 1 |
| | 0 et 2 |
| | 1 et 2 |
| | 1 et 3 |
| | 2 et 3. |

On peut néanmoins constater une tendance à l'augmentation des titres en fonction de l'intensité de la gastrite :
- au niveau de l'atrophie, les moyennes doublent lorsque l'on passe du niveau 1 à 2 et du niveau 2 à 3 pour HspA et pour l'extrait au NOG de la souche aflagellée.
- au niveau de l'inflammation, les moyennes doublent lorsque l'on passe du niveau 1 à 2.

Le nombre d'effectifs dans chaque groupe est relativement faible (toujours <30) pour conclure à des différences statistiquement significatives.

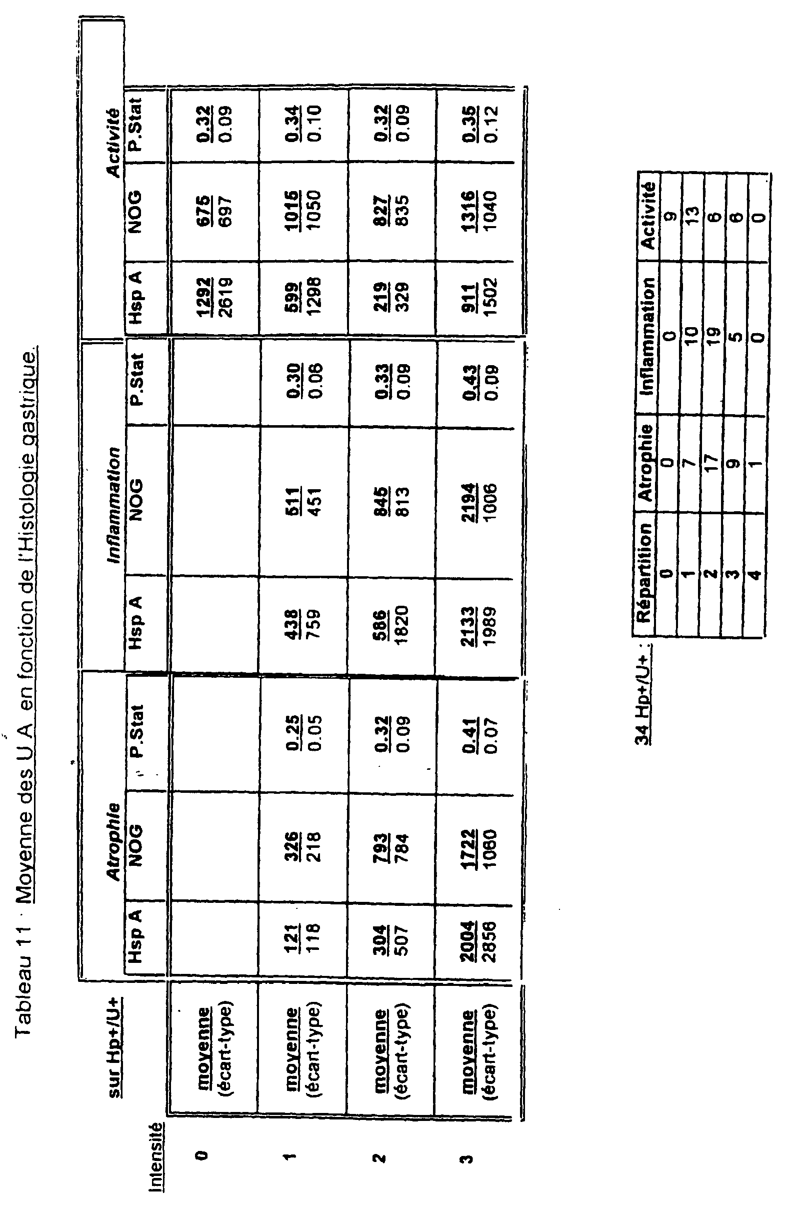

Les sérums pouvant présenter des réactions croisées
2 types de sérums ont été utilisés.
20 sérums (10 anti-Legionella + et 10 anti-Chlamydia +) pouvant présenter des réactions croisées avec HspA, car ces 3 bactéries possèdent des « Heat shock proteins » très voisines les unes des autres.
3 sérums anti-Campylobacter positifs, afin de mettre en évidence des réactions croisées avec la souche flagellée N6, qui disparaîtraient avec la souche aflagellée N6fIbA-. Il est très difficile de se procurer des sérums anti-Camylobacter positifs, c'est pourquoi il n'y a que 3 sérums.
HspA ne présente aucune réaction croisée, ni avec les 10 sérums anti-Legionella positifs, ni avec les 10 sérums anti-Chlamydia positifs.
Certains de ces sérums ont des titres positifs en anticorps anti-Helicobacter pylori, aussi bien avec la souche flagellée qu'avec la souche aflagellée, mais le contexte clinique de ces sérums n'est pas connu.

**Tableau 12 : Sérums pouvant présenter des réactions croisées**

| **Legionella +** | **Titre** | **N6** | **VS=100** | **N6fIBA-** | **VS=60** | **HspA** | **VS=100** |
|---|---|---|---|---|---|---|---|
| A | P2 P3 =256 | 0 | 0 | 4 | 0 | 47 | 0 |
| B | P4 P5 =64 | >928 | 1 | 641 | 1 | 42 | 0 |
| C | P2 P3 =128 | 212 | 1 | 87 | 1 | 68 | 0 |
| D | P2 P3 =64 | 70 | 0 | 19 | 0 | 15 | 0 |
| E | P1=256/P2=512 | >928 | 1 | 239 | 1 | 258 | 1 |
| F | P2 P3 P4 P5 =128 | 322 | 1 | 121 | 1 | 41 | 0 |
| G | P1=512/P6=1024 | >928 | 1 | 193 | 1 | 121 | 1 |
| H | P4 P5=64 | >928 | 1 | 479 | 1 | 18 | 0 |
| I | P2=128/P3=64 | 33 | 0 | 17 | 0 | 25 | 0 |
| J | P2=256/P3=128 | 16 | 0 | 8 | 0 | 32 | 0 |
| | | | | | | | |

| **Chlamydia +** | **Titre** | **N6** | **VS=100** | **N6fIBA-** | **VS=60** | HspA | **VS=100** |
|---|---|---|---|---|---|---|---|
| A | 256 | 6 | 0 | 8 | 0 | 25 | 0 |
| B | 256 | 7 | 0 | 9 | 0 | 34 | 0 |
| C | 64 | 636 | 1 | 290 | 1 | 39 | 0 |
| D | 256 | 367 | 1 | 225 | 1 | 19 | 0 |
| E | 32 | > 928 - | 1 | 855 | 1 | 19 | 0 |
| F | 128 | >928 | 1 | 783 | 1 | 27 | 0 |
| G | 32 | 115 | 1 | 55 | 0 | 15 | 0 |
| H Twar | 16 | 19 | 0 | 10 | 0 | 14 | 0 |
| I | 32 | >928 | 1 | 592 | 1 | >928 | 1 |
| J Twar | 64 | 610 | 1 | 280 | 1 | 44 | 0 |
| | | | | | | | |

| **Campylobacter +** | | **N6** | **VS=100** | **N6fIBA-** | **VS=60** | **HspA** | **VS=100** |
|---|---|---|---|---|---|---|---|
| A | | 35 | 0 | 28 | 0 | 17 | 0 |
| B | | 13 | 0 | 4 | 0 | 27 | 0 |
| C | | 50 | 0 | 68 | 1 | 89 | 0 |

### CONCLUSION

### HspA malE

Cette molécule ne peut toujours pas être utilisée seule car elle manque aussi de sensibilité, mais elle pourrait être intéressante si on l'associe à d'autres molécules.
Elle présente toutefois un risque de réactions croisées, du fait de la conservation importante de ces « Heat shock proteins » entre les différentes espèces bactériennes

### N6flbA-

Ce variant aflagellé parait très intéressant, la sensibilité et la spécificité obtenues avec la population Il de sérums montre une performance très intéresssante.

### N6

Pour l'instant, la souche flagellée semble intéressante. Cependant, les réactions croisées relatives au flagelle n'ont été que peu étudiées en raison de la difficulté à s procurer des sérums bien documentés en sérologie Campylobacter.

### Test JLF

Un test sérologique basé sur l'extraction aqueuse (PBS) de plusieurs souches d'Helicobacter pylori a été mis au point. Ce test semble très performant.
Un extrait NOG du variant aflagellé a été utilisé pour tester la population I de sérums.
87 sérums, documentés du point de vue bactériologique et anatomopathologique uniquement ont été testés avec l'extrait bactérien aflagellé.
Un sérum est positif si la culture est positive, ou si l'anapath, et le test rapide à l'urée sont positifs.
Un sérum est négatif si les 3 tests (culture, anapath, et test rapide à l'urée) sont négatifs.
On trouve une Sensibilité de 90.3% (28/31) pour une Spécificité de 71.4% (40/56).
Sur 16 sérums faux positifs avec un premier test, 9 sont positifs soit avec la sérologie JLF, soit avec le Western Blot JLF, soit avec les deux.
Sur les 3 sérums faux négatifs avec un premier test, les 3 sont négatifs, soit avec la sérologie JLF, soit avec le Western Blot JLF, et un sérum est négatif avec les deux systèmes.

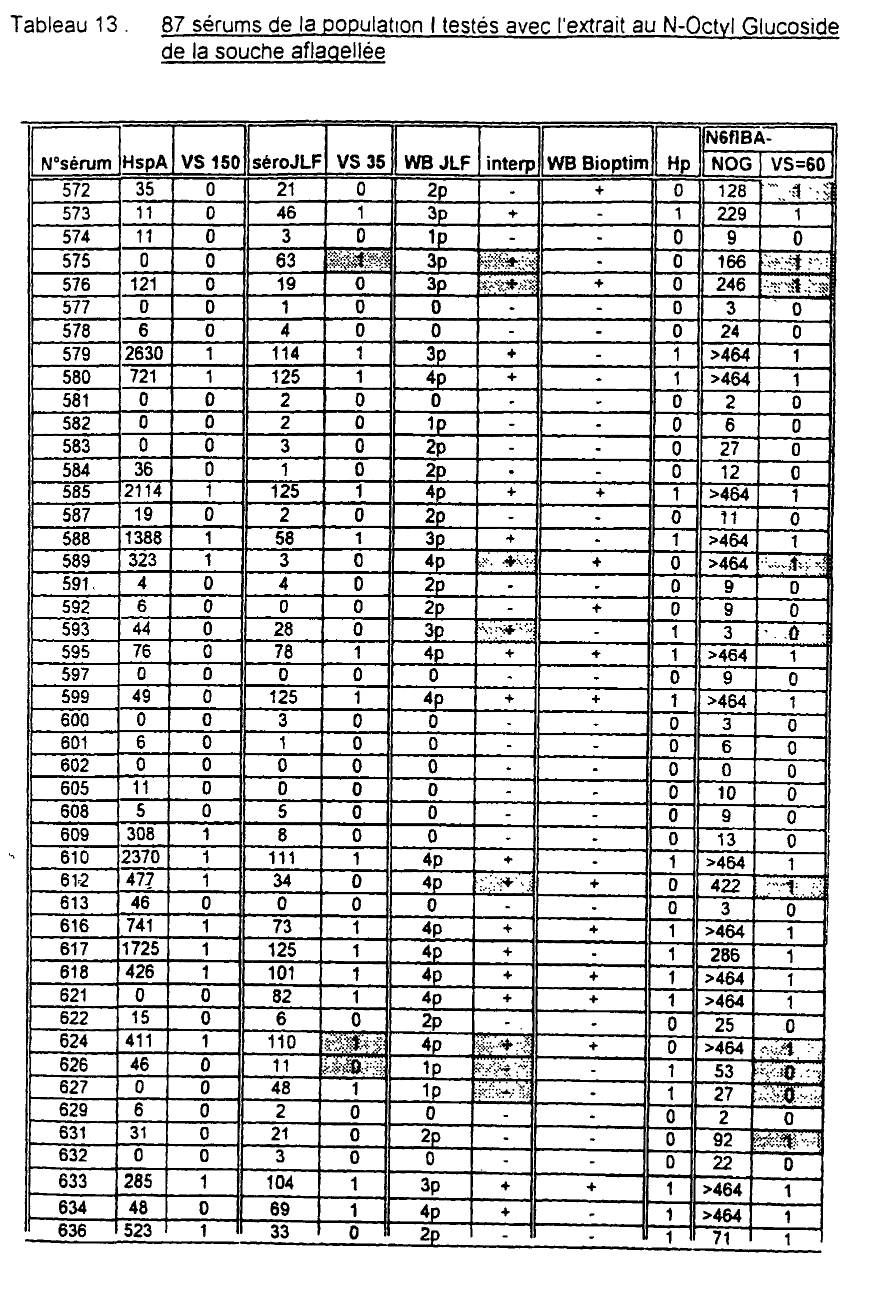

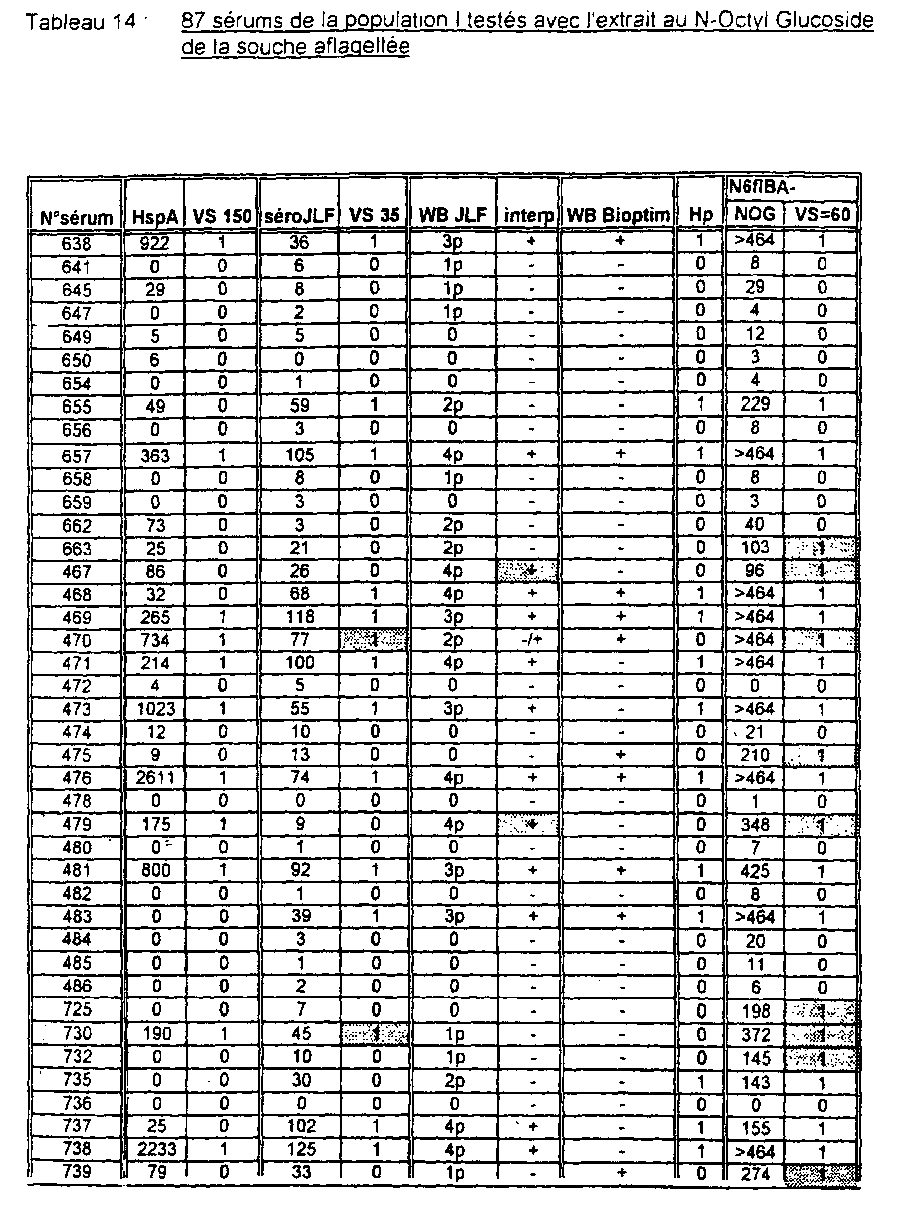

**TECHNIQUE**

| | | | |
|---|---|---|---|
| Plaques coatées à : | 2 µg/ml en Antigène | | Hsp A |
| | 3 µg/ml en extrait | | NOG de NfIbA et N6 |
| Gamme: | 5 points de gamme | | témoin négatif |
| dilutions | | | témoin positif utilisé à 4 |
| Sérums de patients: | dilution au 1/100 | | |
| | volume déposé | | 100µl |
| - Incubation : | 1 heure à 37°C | | |
| - 3 lavages | | | |
| Conjugué monoclonal (IgG toxo) utilisé au | | 1/32000 pour HspA | |
| | | 1/64000 pour N6fIbA- | |
| | | 1/56000 pour N6 | |
| | volume déposé | | 100 µl |

- Incubation du conjugué 1 heure à 37°C
- 4 lavages
- Révélation de la réaction enzymatique par OPD + substrat 30 minutes à l'obscurité
- Arrêt de la réaction enzymatique par H₂SO₄
- Lecture des D.O. à 492 nm / 620 nm

Conversion desD.O en Unités Arbitraires (UA).

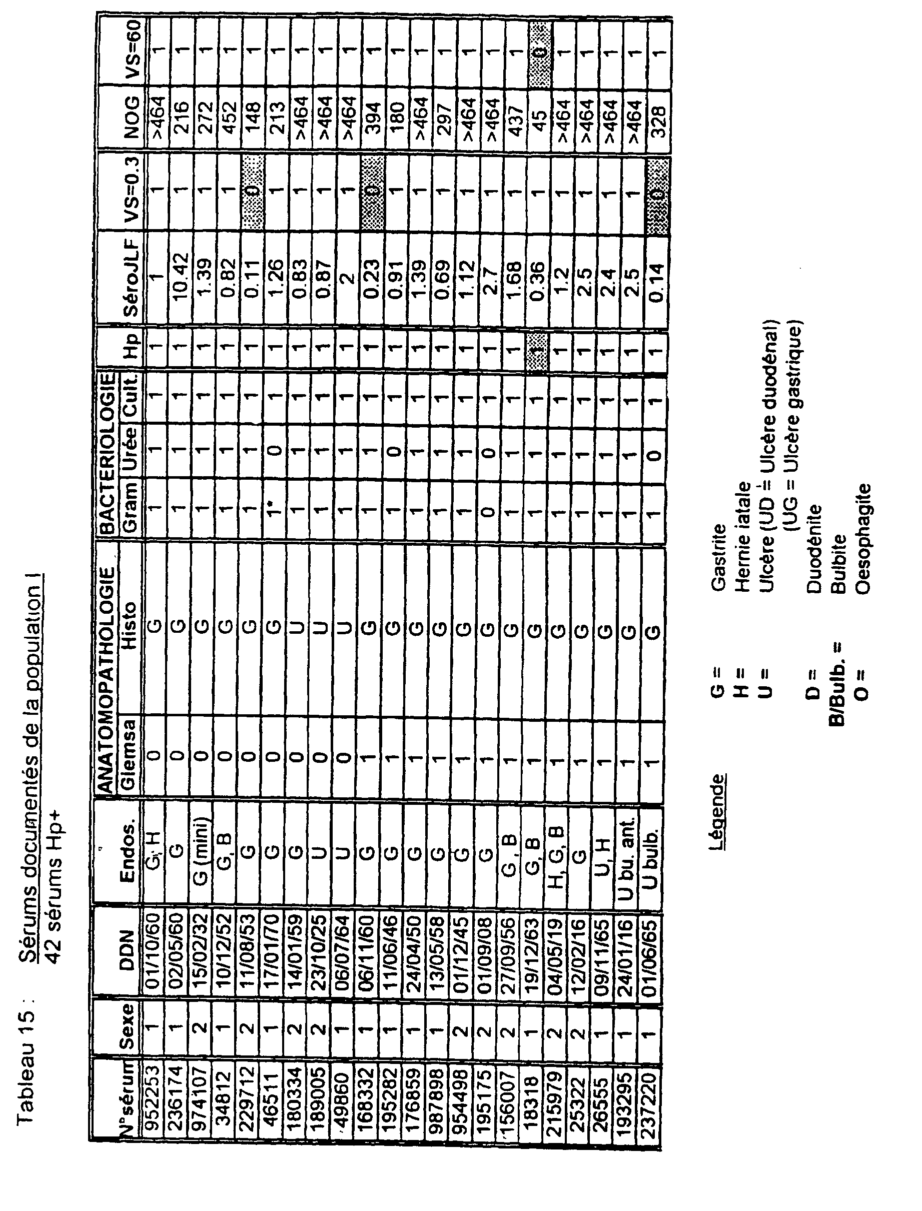

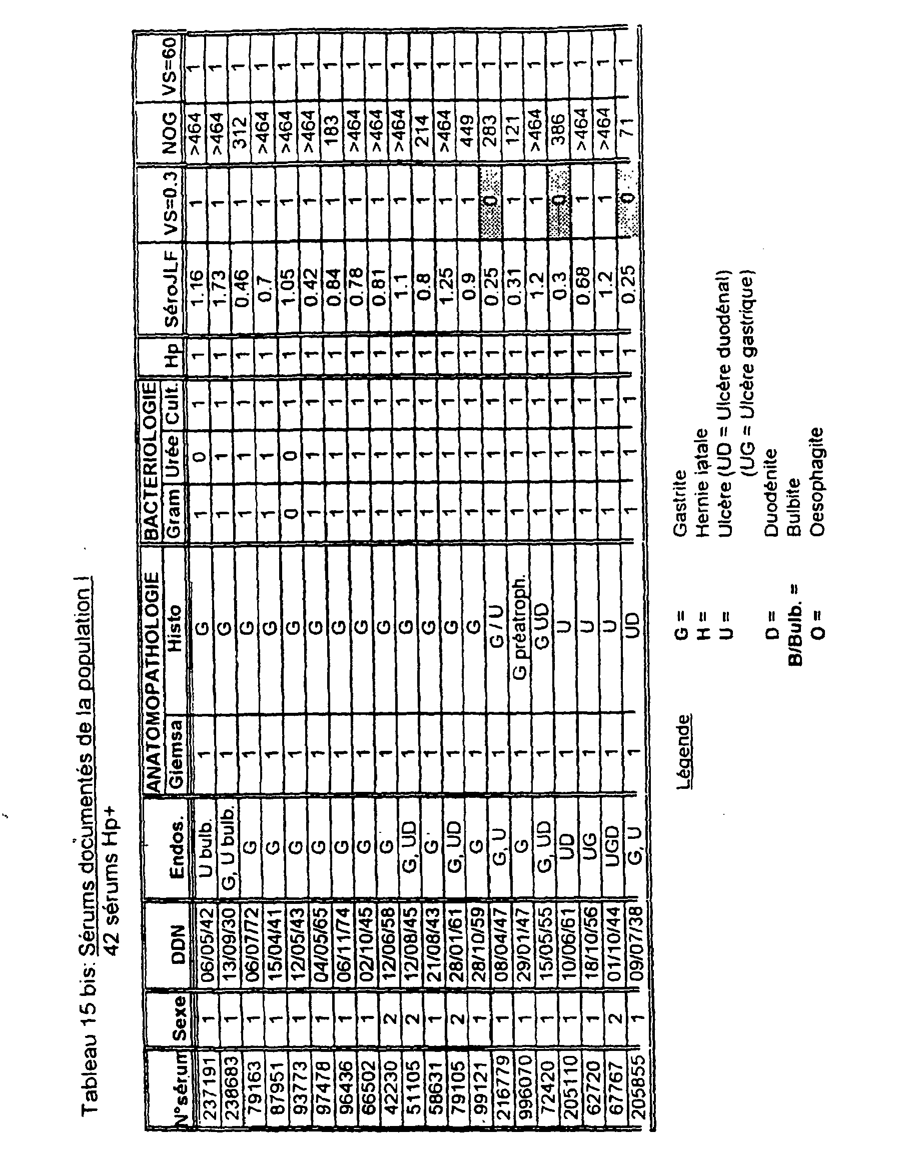

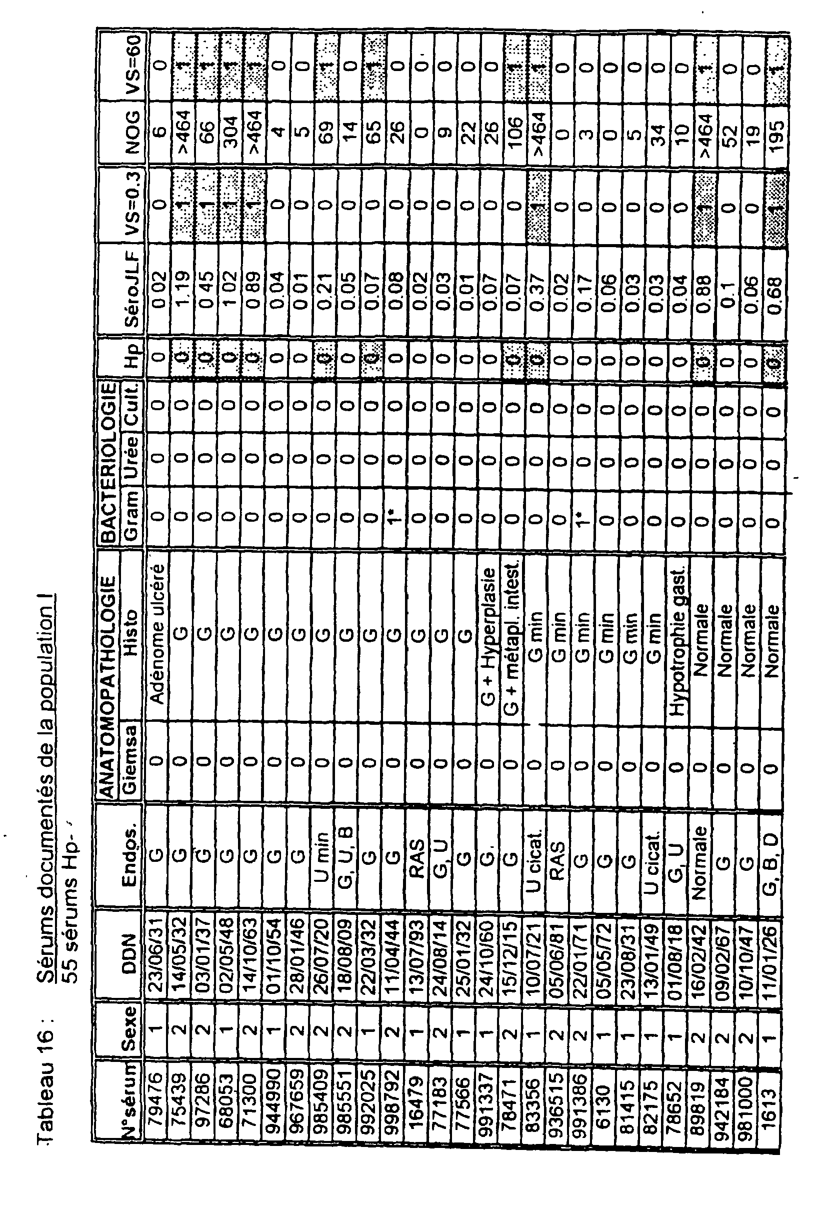

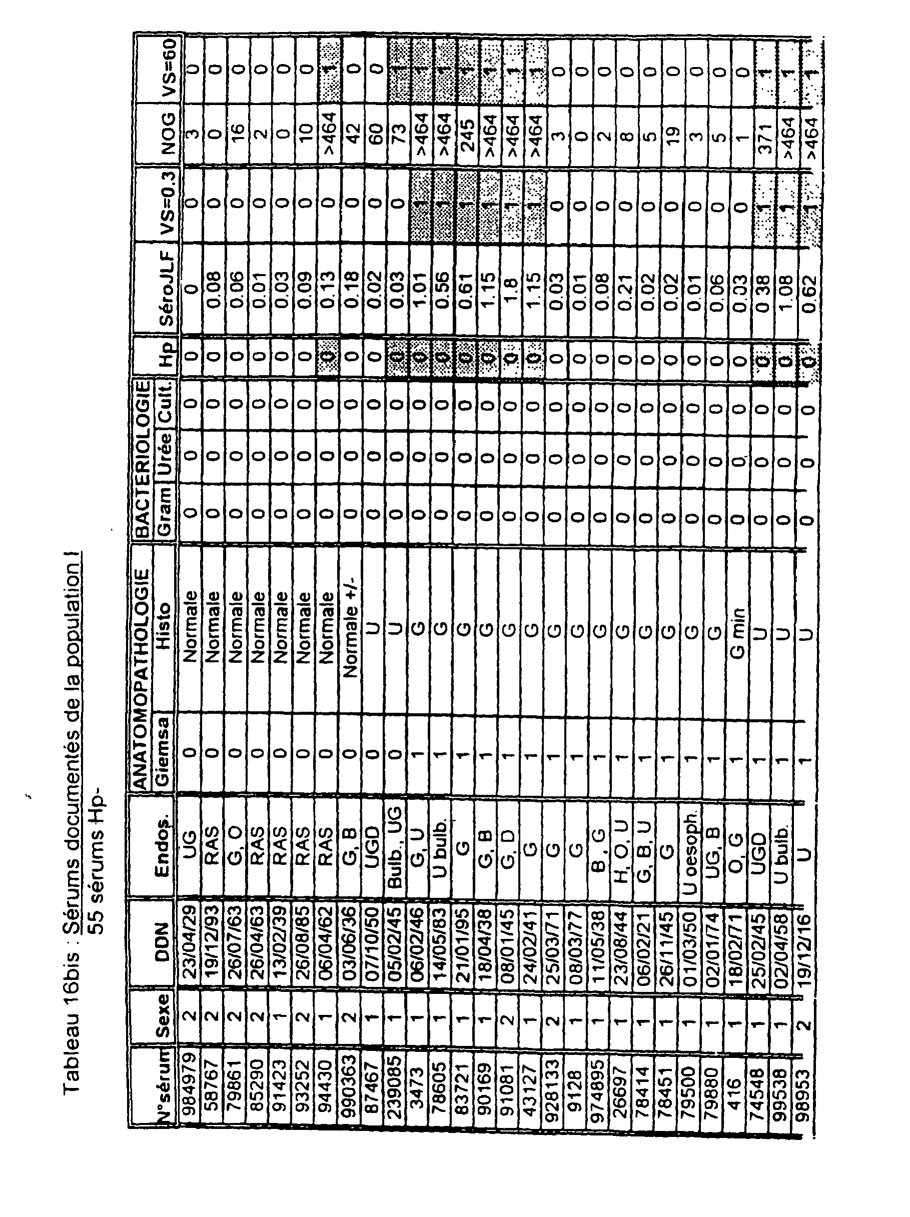

**Tableau 17 : Population documentée de la population I 55 sérums Hp-42 sérums Hp+**

| | **SENSIBILITE** | **SPECIFICITE** |
|---|---|---|
| Séro JLF | 85.7% (36/42) | 70.9% (39/55) |
| NOG 60 | 97.6% (41/42) | 61.8% (34/55) |

### PROTOCOLES D'EXTRACTION A PARTIR DE LA SOUCHE AFFLAGELLEE N6flbA-.

Quantité fournie 800 mg de bactéries recueillies avec du PBS, centrifugées
3 extractions testées.

**EXTRACTIONS DE LA SOUCHE AFLAGELLEE**

| | Extraction Glycine | Extraction N-Octyl Glucoside | Extraction PBS |
|---|---|---|---|
| Récupération | PBS | PBS 0.01 M | PBS pH 7.4 |
| Lavage | 2 fois dans du PBS 8.000 rpm/12 min | 2 fois dans du PBS 8.000 rpm / 12 min | |
| Extraction | Tampon Glycine Acide 0.2M pH 2.2 15 min. Tp ambiante agitation douce 100 mg (poids humide) pour 2.5 ml | PBS contenant : 1% N-Octyl Glucoside pH 7.2 | Vortexer pendant 1 min. |
| | | (Sigma Chemical Co.) 20 min. Tp ambiante | |
| Centrifugation | 11.000 g 15 min. | 23.500 g 20 min. | 5.000 g 10 min. |
| Neutralisation | NaOH 1M | | |
| Dialyse | PBS pH 7.2 24 H à +4°C cut-off 10.000 | PBS pH 7.2 24 H à +4°C cut-off 10.000 | PBS pH 7.2 24 H à +4°C cut-off 10.000 |
| Conservation | détermination de la concentration | élimination des particules insolubles | détermination de la concentration |
| | Conservation à -20°C | conservation à -20°C | Conservation à -20°C |

### SDS PAGE DES DIFFERENTS EXTRAITS DE LA SOUCHE AFLAGELLEE N6 FLBA-

| N° puits | Type d'échantillon | Concentration ug/ml | Volume échantillon / Volume à Tampon | Volume déposé |
|---|---|---|---|---|
| 1 | Etalon PM | | 5 + 5 / 190 | 10 |
| 2 | Extrait Glycine | 202.9 | 60/ 60 | 60 |
| 3 | | | | |
| 4 | Extrait N-Octyl Glucoside | 874 | 51 / 39 | 60 |
| 5 | | | | |
| 6 | Extrait PBS I | 539.2 | 60/20 | 60 |
| 7 | | | | |
| 8 | Extrait PBS 2 | 77.9 | 60 / 20 | 60 |
| 9 | | | | |
| 10 | Etalon PM | | 5 + 5 / 190 | 10 |
| 11 | Culot Extrait Glycine | 2778.7 | 20/20 | 20 |
| 12 | | | | |
| 13 | Culot Extrait Glucoside | 972.9 | 40/40 | 60 |
| 14 | | | | |
| 15 | Extrait Glycine sédimenté | 309.3 | 60/20 | 60 |
| 16 | | | | |
| 17 | HspA Mal E | 3000 | 20/20 | 20 |
| 18 | | | | |
| 19 | | | | |
| 20 | Kaléidoscope | | | 20 |

### Références :

1. Andrews, G.P., Maurelli, A.T. : mxiA of Shigella flexneri 2a, which facilitates export of invasion plasmid antigens, encodes a homolog of the low-calcium-response protein. LcrD of Yersinia pestis. Infect. Immun. 60: 3287-3295 (1992).
2. Galan, J.E., Ginocchio, C. Costeas, P. : Molecular and functional characterization of the Salmonella invasion gene invA : homology of InvA to members of a new protein family. J. Bacteriol. 174, 4338-4349 (1992.
3. Leying, H., Suerbaum, S. Geis, G., Haas, R. : Cloning and genetic characterization of a Helicobacter pylori flagellin gene. Mol. Microbiol. 6. 2563-2874 (1993).
5. O'Toole, P.W., Kostrzynska, M., Trust, T.J.: Non-mobile mutants of Helicobacter pylori and Helicobacter mustelae defective in flagellar hook production. Mol. Microbiol. 14, 691-703 (1994).
6. Plano, G.V., Barve, S.S., Straley, S.C. : LcrD, a membrane-bound regulator of the Yersinia pestis low-calcium response. J. Bacteriol. 173. 7293-7303 (1991).
7. Ramakrishnaan, G., Zhao, J-L., Newton, A. : The cell cycle-regulated gene flbf of Caulobacter crescentus is homologous to a virulence locus of Yersinia pestis. J. Bacteriol. 173, 7283-7292 (1991).
8. Suerbaum, S., Josenhans, C., Labigne, A.: Cloning and genetic characterization of the Helicobacter pylori and Helicobacter mustelae flab flagellin genes and construction of H. pylori flaA- and flaB-negative mutants by electroporation-mediated allelic exchange, J. Bacteriol. 175, 3278-3288 (1993).

## Revendications

1. Séquence nucléotidique régulant la biosynthèse des protéines des flagelles d'Helicobacter pylori, capable d'hybrider dans des conditions de forte stringence avec une sonde correspondant à un fragment de nucléotides de H. pylori amplifié à l'aide de deux oligonucléotides répondant aux séquences suivantes :
OLFIbA-1 : ATGCCTCGAGGTCGAAAAGCAAGATG
OLFIbA-2 : GAAATCTTCATACTGGCAGCTCCAGTC, ou capable d'hybrider dans des conditions de forte stringence avec ces oligonucléotides.

2. Séquence nucléotidique du gène flbA régulant la biosynthèse des protéines des flagelles d'Helicobacter pylori, telle qu'obtenue par les étapes de :
- criblage d'une banque génomique contenant l'ADN chromosomique d'une souche H. pylori avec une sonde correspondant à un fragment de nucléotides de H pylori amplifié à l'aide de deux nucléotides répondant aux séquences suivantes :
OLFIbA-1 : ATGCCTCGAGGTCGAAAAGCAAGATG
OLFIbA-2 : GAAATCTTCATACTGGCAGCTCCAGTC ou capable d'hybrider dans des conditions de forte stringence avec ces oligonucléotides
- récupération des séquences d'ADN hybridant avec ladite sonde,
- sous-clonage des séquences d'ADN obtenus dans un vecteur approprié de type plasmide et sélection de ceux des vecteurs modifiés hybridant dans des conditions de forte stringence avec la sonde correspondant au fragment d'ADN de H pylori amplifié à l'aide des oligonucléotides OLFIbA-1 et OLFIbA-2,
- séquençage des fragments d'ADN contenus dans les vecteurs plasmidiques hybridant avec la susdite sonde et détermination de la phase ouverte de lecture contenue dans ces fragments.

3. Séquence nucléotidique selon la revendication 1 ou la revendication 2, **caractérisée en ce qu'**elle répond à l'enchaînement de nucléotides représenté à la figure 2.

4. Séquence nucléotidique selon l'une quelconque des revendications 1 à 3, **caractérisée en ce que** qu'elle code pour une protéine répondant à la séquence d'acides aminés représentée à la figure 2 ou pour une séquence d'acides aminés ayant les mêmes propriétés régulatrices vis à vis de la biosynthèse des protéines des flagelles de H. pylori, que la susdite séquence.

5. Séquence nucléotidique selon l'une quelconque des revendications 1 à 5, modifiée par délétion, substitution ou insertion de bases ou d'un fragment d'une séquence de nucléotides de façon telle que le gène **flbA** n'est plus exprimé dans un hôte cellulaire ou de façon telle que l'expression du gène **flbA** dans un hôte cellulaire ne permet pas la biosynthèse des flagelles de H pylori et le cas échéant ne permet pas la synthèse de la protéine du crochet de H. pylori.

6. Séquence nucléotidique correspondant à un fragment du gène flbA selon l'une quelconque des revendications 1 à 4, **caractérisée en ce qu'**il s'agit d'un fragment d'au moins 6 nucléotides, de préférence d'au moins 100 nucléotides issu du gène flbA, de préférence délimité par des sites de restriction, présents dans la séquence du gène flbA.

7. Acide nucléique recombinant, **caractérisé en ce qu'**il comprend une séquence nucléotidique selon l'une quelconque des revendications 1 à 6 modifiée par insertion d'une cassette contenant un marqueur, par exemple un gène de résistance à un antibiotique, ou un gène de résistance à un métal lourd.

8. Acide nucléique recombinant selon la revendication 7, **caractérisé en ce que** la séquence nucléotidique selon l'une quelconque des revendications 1 à 6 est modifiée par insertion d'une cassette de résistance à la kanamycine.

9. Oligonucléotides **caractérisés en ce qu'**ils sont spécifiques d'une séquence selon l'une quelconque des revendications 1 à 3, et **en ce qu'**ils répondent à l'une des séquences suivantes :
OLFIbA-1: ATGCCTCGAGGTCGAAAAGCAAGATG
OLFIbA-2: GAAATCTTCATACTGGCAGCTCCAGTC
OLFlbA-7: CGGGATCCGTGGTTACTAATGGTTCTAC
OLFIbA-8: CGGGATCCTCATGGCCTCTTCAGAGACC

10. Séquence d'acides aminés de la protéine FlbA de H. pylori, **caractérisée en ce qu'**elle est codée par une séquence nucléotidique selon l'une quelconque des revendications 1 ou 2.

11. Séquence d'acides aminés **caractérisée en ce qu'**il s'agit de la protéine FlbA de H. Pylori, répondant à l'enchaînement présenté à la figure 2, ou **en ce qu'**il s'agit d'un fragment de cette protéine dès lors qu'il est reconnu par des anticorps dirigés contre la protéine FlbA.

12. Souche bactérienne d'Helicobacter pylori, **caractérisée en ce qu'**elle a un phénotype aflagellé résultant de la mutation par substitution, addition et/ou de la délétion de bases ou d'un fragment nucléotidique, la séquence nucléotidique selon l'une quelconque des revendications 1 à 3 du gène flbA participant à la régulation de la biosynthèse des protéines des flagelles de H. pylori.

13. Souche bactérienne selon la revendication 12, **caractérisée en ce qu'**elle est en outre dépourvue de la protéine du crochet de H. pylori.

14. Souche bactérienne recombinante selon la revendication 12 ou la revendication 13, **caractérisée en ce qu'**elle est obtenue à partir de la souche N6 déposée à la NCIMB le 26 Juin 1992 sous le numéro NCIMB 40512.

15. Souche bactérienne recombinante selon l'une quelconque des revendications 12 ou 14, **caractérisée en ce qu'**il s'agit de la souche N6flbA- déposée à la NCIMB le 30 Juin 1995 sous le numéro NCIMB 40747.

16. Souche bactérienne selon l'une quelconque des revendications 12 à 15, **caractérisée en ce qu'**elle est en outre mutée de façon à produire une uréase atténuée, voire à ne plus produire d'uréase, la mutation consistant par exemple en une mutation de la séquence nucléotidique d'un ou plusieurs gènes choisis parmi les gènes ureA, ureB, ureC, ureD, ureE, uref, ureG, ureH ou urel.

17. Extrait bactérien **caractérisé en ce que** qu'il s'agit d'un extrait de souches bactériennes selon l'une quelconque des revendications 12 à 15.

18. Extrait bactérien selon la revendication 17, **caractérisé en ce qu'**il est obtenu après une extraction au N-OctylGlucoside.

19. Extrait bactérien selon la revendication 17, **caractérisé en ce qu'**il est obtenu après une extraction au PBS ou à la glycine.

20. Composition pour la détection in vitro d'une infection par H. pylori dans un échantillon de fluide biologique chez un patient, notamment dans un échantillon de sérum, comprenant à titre de principe actif, une souche bactérienne selon l'une quelconque des revendications 12 à 15 ou un extrait bactérien selon l'une quelconque des revendications 17 à 19.

21. Procédé de détection in vitro d'une infection par H. pylori dans un échantillon de fluide biologique chez un patient, notamment dans un échantillon de sérum, comprenant les étapes de :
- mise en contact de l'échantillon testé avec une souche bactérienne selon l'une quelconque des revendications 12 à 15, ou avec un extrait bactérien selon l'une quelconque des revendications 17 à 19,
- détection d'une réaction immunologique entre ladite souche bactérienne et des anticorps dirigés contre H. pylori, présents dans l'échantillon testé.

22. Composition immunogène pour l'obtention d'anticorps contre H. pylori, **caractérisée en ce qu'**elle comprend à titre de principe actif, une souche bactérienne selon l'une quelconque des revendications 12 à 16 ou un extrait bactérien selon l'une quelconque des revendications 17 à 19.

23. Composition immunogène, pour l'obtention d'anticorps contre H. pylori, **caractérisée en ce qu'**elle comprend une séquence d'acides aminés selon l'une quelconque des revendications 10 ou 11.

24. Composition vaccinante pour l'obtention d'anticorps protecteurs contre une infection par H. pylori, **caractérisée en ce qu'**elle comprend à titre de principe actif, une souche bactérienne selon l'une quelconque des revendications 12 à 16 ou un extrait bactérien selon l'une quelconque des revendications 17 à 19.

25. Composition vaccinante pour l'obtention d'anticorps contre une infection par H. pylori, **caractérisée en ce qu'**elle comprend à titre de principe actif, des antigènes de type uréase ou participant à l'activité uréase de H. pylori, en particulier des antigènes codés par les gènes ureA, ureB, ureC ou ureD et une protéine répondant à une séquence d'acides aminés selon l'une quelconque des revendications 10 ou 11.

26. Anticorps monoclonaux ou sérum polyclonal dirigés contre une séquence d'acides aminés selon l'une quelconque des revendications 10 ou 11.

27. Anticorps monoclonaux ou sérum polyclonal dirigé contre une souche de H. pylori selon l'une quelconque des revendications 12 à 15.

28. Composition pour la détection in vitro d'une infection par H. pylori dans un échantillon biologique, comprenant à titre de principe actif des anticorps monoclonaux ou un sérum polyclonal obtenus contre une souche de H. pylori de phénotype aflagellé selon l'une quelconque des revendications 12 à 15.

29. Utilisation des séquences nucléotidiques selon l'une quelconque des revendications 1 à 9, pour la préparation de compositions immunogènes pour l'obtention d'anticorps contre H. pylori.

30. Coffret de diagnostic d'anticorps de patients infectés par H. pylori comprenant un extrait bactérien selon l'une quelconque des revendications 15 à 19, et des réactifs nécessaires pour mettre en évidence un réaction de type antigène/anticorps.


LISTE DE SEQUENCES

(1) INFORMATIONS GENERALES:

(i) DEPOSANT:
(A) NOM: INSTITUT PASTEUR
(B) RUE: 25-28 rue du Dr. Roux
(C) VILLE: PARIS
(E) PAYS: FRANCE
(F) CODE POSTAL: 75724 CEDEX 15

(A) NOM: INSTITUT NATIONAL DE LA SANTE ET DE LA
RECHERCHE MEDICALE
(B) RUE: 101 rue de Tolbiac
(C) VILLE: PARIS
(E) PAYS: FRANCE
(F) CODE POSTAL: 75654 CEDEX 13

(ii) TITRE DE L' INVENTION: CLONAGE ET CARACTERISATION DU GENE flbA de
H.
PYLORI, PRODUCTION DE SOUCHES AFLAGELLEES

(iii) NOMBRE DE SEQUENCES: 12

(iv) FORME DECHIFFRABLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(v) DONNEES DE LA DEMANDE ACTUELLE:
NUMERO DE LA DEMANDE: EP 96401445.0
(vi) DONNEES DE LA DEMANDE ANTERIEURE:
(A) NUMERO DE LA DEMANDE: FR 9508068
(B) DATE DE DEPOT: 04-JUL-1995

(2) INFORMATIONS POUR LA SEQ ID NO: 1:

(i) CARACTERISTIQUES DE LA SEQUENCE:
(A) LONGUEUR: 26 paires de bases
(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "OLIGONUCLEOTIDE"

(xi) DESCRIPTION DE LA SEQUENCE: SEQ ID NO: 1:

ATGCCTCGAG GTCGAAAAGC AAGATG 26

(2) INFORMATIONS POUR LA SEQ ID NO: 2:

(i) CARACTERISTIQUES DE LA SEQUENCE:
(A) LONGUEUR: 27 paires de bases
(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "OLIGONUCLEOTIDE"

(xi) DESCRIPTION DE LA SEQUENCE: SEQ ID NO: 2:

GAAATCTTCA TACTGGCAGC TCCAGTC 27

(2) INFORMATIONS POUR LA SEQ ID NO: 3:

(i) CARACTERISTIQUES DE LA SEQUENCE:
(A) LONGUEUR: 135 paires de bases
(B) TYPE: nucléotide
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 3:

ATGCCAGGAA AGCAAATGGC GATTGATGCG GATTTAAATT CAGGGCTTAT TGATGATAAG 60

GAAGCTAAAA AACGGCGCGC CGCTCTAAGC CAAGAAGCGG ATTTTTATGG TGCGATGGAT 120

GGCGCGTCTA AATTT 135

(2) INFORMATIONS POUR LA SEQ ID NO: 4:

(i) CARACTERISTIQUES DE LA SEQUENCE:
(A) LONGUEUR: 28 paires de bases
(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "OLIGONUCLEOTIDE"

(xi) DESCRIPTION DE LA SEQUENCE: SEQ ID NO: 4:

CGGGATCCGT GGTTACTAAT GGTTCTAC 28

(2) INFORMATIONS POUR LA SEQ ID NO: 5:

(i) CARACTERISTIQUES DE LA SEQUENCE:
(A) LONGUEUR: 28 paires de bases
(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "OLIGONUCLEOTIDE"

(xi) DESCRIPTION DE LA SEQUENCE: SEQ ID NO: 5:

CGGGATCCTC ATGGCCTCTT CAGAGACC 28

(2) INFORMATIONS POUR LA SEQ ID NO: 6:

(i) CARACTERISTIQUES DE LA SEQUENCE:
(A) LONGUEUR: 2501 paires de bases
(B) TYPE: nucléotide
(C) NOMBRE DE BRINS: simple
(D) CONFIGURATION: linéaire

(ii) TYPE DE MOLECULE: ADN (génomique)

(ix) CARACTERISTIQUE:
(A) NOM/CLE: CDS
(B) EMPLACEMENT:85..2280
(D) AUTRES INFORMATIONS:/note= "SEQUENCE NUCLEOTIDIQUE DU
GENE flbA de H. pylori ET SEQUENCE EN ACIDES AMINES DEDUITE"

(xi) DESCRIPTION DE LA SEQUENCE: SEQ ID NO: 6:
   AGCTTTTTTG TGCCATACTT TTAAACTTTA TATTATAATA AGAGACAAAC ACACCTACCA 60
   AAATTAAGGC ATTGATTTTA GATT ATG GCA AAC GAA CGC TCC AAA TTA GCT 111
   Met Ala Asn Glu Arg Ser Lys Leu Ala
   1 5
   TTT AAA AAG ACT TTC CCT GTC TTT AAA CGC TTC TTG CAA TCC AAA GAC 159
   Phe Lys Lys Thr Phe Pro Val Phe Lys Arg Phe Leu Gln Ser Lys Asp
   10 15 20 25
   TTA GCC CTT GTG GTC TTT GTG ATA GCG ATT TTA GCG ATC ATT ATC GTG 207
   Leu Ala Leu Val Val Phe Val Ile Ala Ile Leu Ala Ile Ile Ile Val
   30 35 40
   CCG TTA CCG CCT TTT GTG TTG GAT TTT TTA CTC ACG ATT TCT ATC GCG 255
   Pro Leu Pro Pro Phe Val Leu Asp Phe Leu Leu Thr Ile Ser Ile Ala
   45 50 55
   CTA TCG GTG TTG ATT ATT TTA ATC GGG CTT TAT ATT GAC AAA CCG ACT 303
   Leu Ser Val Leu Ile Ile Leu Ile Gly Leu Tyr Ile Asp Lys Pro Thr
   60 65 70
   GAT TTT AGC GCT TTC CCC ACT TTA TTA CTC ATT GTA ACC TTA TAC CGC 351
   Asp Phe Ser Ala Phe Pro Thr Leu Leu Leu Ile Val Thr Leu Tyr Arg
   75 80 85
   TTG GCT TTA AAT GTC GCC ACC ACT AGA ATG ATT TTA ACC CAA GGC TAT 399
   Leu Ala Leu Asn Val Ala Thr Thr Arg Met Ile Leu Thr Gln Gly Tyr
   90 95 100 105
   AAA GGG CCT AGC GCG GTG AGC ATT ATT ATC ACG GCG TTT GGG GAA TTT 447
   Lys Gly Pro Ser Ala Val Ser Ile Ile Ile Thr Ala Phe Gly Glu Phe
   110 115 120
   AGC GTG AGC GGG AAT TAT GTG ATT GGG GCT ATT ATC TTT AGT ATT TTA 495
   Ser Val Ser Gly Asn Tyr Val Ile Gly Ala Ile Ile Phe Ser Ile Leu
   125 130 135
   GTG CTG GTG AAT TTA TTA GTG GTT ACT AAT GGT TCT ACT AGG GTT ACT 543
   Val Leu Val Asn Leu Leu Val Val Thr Asn Gly Ser Thr Arg Val Thr
   140 145 150
   GAA GTT AGG GCG CGA TTT GCC CTA GAC GCT ATG CCA GGA AAG CAA ATG 591
   Glu Val Arg Ala Arg Phe Ala Leu Asp Ala Met Pro Gly Lys Gln Met
   155 160 165
   GCG ATT GAT GCG GAT TTA AAT TCA GGG CTT ATT GAT GAT AAG GAA GCT 639
   Ala Ile Asp Ala Asp Leu Asn Ser Gly Leu Ile Asp Asp Lys Glu Ala
   170 175 180 185
   AAA AAA CGG CGC GCC GCT CTA AGC CAA GAA GCG GAT TTT TAT GGT GCG 687
   Lys Lys Arg Arg Ala Ala Leu Ser Gln Glu Ala Asp Phe Tyr Gly Ala
   190 195 200
   ATG GAT GGC GCG TCT AAA TTT GTC AAA GGC GAT GCG ATC GCT TCT ATC 735
   Met Asp Gly Ala Ser Lys Phe Val Lys Gly Asp Ala Ile Ala Ser Ile
   205 210 215
   ATT ATC ACG CTT ATC AAT ATC ATT GGG GGT TTT TTA GTG GGC GTG TTC 783
   Ile Ile Thr Leu Ile Asn Ile Ile Gly Gly Phe Leu Val Gly Val Phe
   220 225 230
   CAA AGG GAT ATG AGC TTG AGC TTT AGT GCT AGC ACT TTC ACT ATC TTA 831
   Gln Arg Asp Met Ser Leu Ser Phe Ser Ala Ser Thr Phe Thr Ile Leu
   235 240 245
   ACC ATT GGC GAT GGG CTT GTA GGG CAA ATC CCT GCC TTA ATC ATT GCG 879
   Thr Ile Gly Asp Gly Leu Val Gly Gln Ile Pro Ala Leu Ile Ile Ala
   250 255 260 265
   ACA CGG ACC GGT ATT GTC GCC ACT CGC ACC ACG CAA AAC GAA GAA GAG 927
   Thr Arg Thr Gly Ile Val Ala Thr Arg Thr Thr Gln Asn Glu Glu Glu
   270 275 280
   GAC TTT GCT TCT AAG CTC ATC ACA CAG CTC ACC AAT AAA AGC AAA ACT 975
   Asp Phe Ala Ser Lys Leu Ile Thr Gln Leu Thr Asn Lys Ser Lys Thr
   285 290 295
   TTA GTG ATT GTG GGG GCG ATT TAT TGC TTT TGC ACC ATT CCT GGA CTC 1023
   Leu Val Ile Val Gly Ala Ile Tyr Cys Phe Cys Thr Ile Pro Gly Leu
   300 305 310
   CCT ACC TTT TCT TTA GCG TTT GTA GGG GCT CTC TTT TTA TTC ATC GCA 1071
   Pro Thr Phe Ser Leu Ala Phe Val Gly Ala Leu Phe Leu Phe Ile Ala
   315 320 325
   TGG CTG ATT AGC AGG GAG GGA AAG GAC GGG TTG CTC ACT AAA TTA GAA 1119
   Trp Leu Ile Ser Arg Glu Gly Lys Asp Gly Leu Leu Thr Lys Leu Glu
   330 335 340 345
   AAT TAT TTG AGT CAA AAA TTC GGC TTG GAT TTG AGC GAA AAA CCC CAC 1167
   Asn Tyr Leu Ser Gln Lys Phe Gly Leu Asp Leu Ser Glu Lys Pro His
   350 355 360
   AGC TCC AAA ATC AAA CCC CAC GCC CCC ACC ACA AGG GCT AAA ACC CAA 1215
   Ser Ser Lys Ile Lys Pro His Ala Pro Thr Thr Arg Ala Lys Thr Gln
   365 370 375
   GAA GAG ATT AAA AGA GAA GAA GAG CAA GCC ATT GAT GAA GTG TTA AAA 1263
   Glu Glu Ile Lys Arg Glu Glu Glu Gln Ala Ile Asp Glu Val Leu Lys
   380 385 390
   ATT GAA TTT TTA GAA TTG GCT TTA GGC TAT CAG CTC TAC AGC TTA GCG 1311
   Ile Glu Phe Leu Glu Leu Ala Leu Gly Tyr Gln Leu Tyr Ser Leu Ala
   395 400 405
   GAC ATG AAA CAA GGG GGC GAT TTG TTA GAA AGG ATT AGG GGT ATT AGA 1359
   Asp Met Lys Gln Gly Gly Asp Leu Leu Glu Arg Ile Arg Gly Ile Arg
   410 415 420 425
   AAA AAG ATA GCG AGC GAT TAT GGT TTT TTG ATG CCT CAA ATT AGG ATT 1407
   Lys Lys Ile Ala Ser Asp Tyr Gly Phe Leu Met Pro Gln Ile Arg Ile
   430 435 440
   AGG GAT AAT TTA CAA CTC CCC CCA ACG CAT TAT GAA ATC AAG CTT AAG 1455
   Arg Asp Asn Leu Gln Leu Pro Pro Thr His Tyr Glu Ile Lys Leu Lys
   445 450 455
   GGC ATT GTG ATT GGT GAA GGC ATG GTG ATG CCG GAT AAG TTT TTA GCC 1503
   Gly Ile Val Ile Gly Glu Gly Met Val Met Pro Asp Lys Phe Leu Ala
   460 465 470
   ATG AAT ACC GGT TTT GTG AAT AAA GAA ATT GAA GGC ATT CCT ACT AAA 1551
   Met Asn Thr Gly Phe Val Asn Lys Glu Ile Glu Gly Ile Pro Thr Lys
   475 480 485
   GAG CCG GCT TTT GGA ATG GAC GCT TTA TGG ATT GAA ACT AAA AAT AAA 1599
   Glu Pro Ala Phe Gly Met Asp Ala Leu Trp Ile Glu Thr Lys Asn Lys
   490 495 500 505
   GAA GAA GCC ATC ATT CAA GGC TAT ACC ATT ATT GAT CCA AGC ACC GTT 1647
   Glu Glu Ala Ile Ile Gln Gly Tyr Thr Ile Ile Asp Pro Ser Thr Val
   510 515 520
   ATT GCG ACG CAC ACC AGC GAA TTA GTG AAA AAA TAC GCT GAA GAT TTT 1695
   Ile Ala Thr His Thr Ser Glu Leu Val Lys Lys Tyr Ala Glu Asp Phe
   525 530 535
   ATC ACT AAA GAT GAA GTG AAA TCC CTT TTA GAG CGC TTG GCC AAA GAC 1743
   Ile Thr Lys Asp Glu Val Lys Ser Leu Leu Glu Arg Leu Ala Lys Asp
   540 545 550
   TAT CCT ACG ATT GTA GAA GAG AGT AAA AAA ATC CCC ACC GGT GCG ATC 1791
   Tyr Pro Thr Ile Val Glu Glu Ser Lys Lys Ile Pro Thr Gly Ala Ile
   555 560 565
   CGA TCA GTC TTG CAA GCC TTG TTG CAT GAA AAA ATC CCC ATT AAA GAC 1839
   Arg Ser Val Leu Gln Ala Leu Leu His Glu Lys Ile Pro Ile Lys Asp
   570 575 580 585
   ATG CTC ACT ATT TTA GAA ACG ATT ACC GAT ATT GCG CCA TTA GTT CAA 1887
   Met Leu Thr Ile Leu Glu Thr Ile Thr Asp Ile Ala Pro Leu Val Gln
   590 595 600
   AAC GAT GTG AAT ATC TTA ACC GAA CAA GTG AGG GCG AGG CTT TCT AGG 1935
   Asn Asp Val Asn Ile Leu Thr Glu Gln Val Arg Ala Arg Leu Ser Arg
   605 610 615
   GTG ATC ACT AAC GCT TTT AAA TCT GAA GAC GGG CGT TTG AAA TTT TTA 1983
   Val Ile Thr Asn Ala Phe Lys Ser Glu Asp Gly Arg Leu Lys Phe Leu
   620 625 630
   ACC TTT TCT ACC GAT AGC GAA CAA TTT TTG CTT AAT AAA TTG CGA GAA 2031
   Thr Phe Ser Thr Asp Ser Glu Gln Phe Leu Leu Asn Lys Leu Arg Glu
   635 640 645
   AAT GGC ACT TCT AAG AGC CTA CTA CTC AAT GTG GGC GAA TTG CAA AAA 2079
   Asn Gly Thr Ser Lys Ser Leu Leu Leu Asn Val Gly Glu Leu Gln Lys
   650 655 660 665
   CTC ATT GAA GCG GTC TCT GAA GAG GCC ATG AAA GTC TTG CAA AAA GGG 2127
   Leu Ile Glu Ala Val Ser Glu Glu Ala Met Lys Val Leu Gln Lys Gly
   670 675 680
   ATC GCT CCG GTG ATT TTG ATC GTA GAG CCT AAT TTA AGA AAA GCC CTT 2175
   Ile Ala Pro Val Ile Leu Ile Val Glu Pro Asn Leu Arg Lys Ala Leu
   685 690 695
   TCT AAT CAA ATG GAG CAG GCT AGG ATT GAT GTA ATC GTG CTA AGC CAT 2223
   Ser Asn Gln Met Glu Gln Ala Arg Ile Asp Val Ile Val Leu Ser His
   700 705 710
   GCT GAA TTA GAT CCT AAC TCT AAT TTT GAA GCC TTA GGC ACG ATC CAT 2271
   Ala Glu Leu Asp Pro Asn Ser Asn Phe Glu Ala Leu Gly Thr Ile His
   715 720 725
   ATT AAC TTT TAAGGGATAA ATAATTGATA AAAAAGGAGA ATGATGCAAG 2320
   Ile Asn Phe
   730
   TTTATCACCT TTCACACATT GATTTAGACG GCTATGCATG CCAGCTTGTT TCAAAACAAT 2380
   TTTTTAAAAA TATCCAATGC TATAACGCTA ATTACGGGCG TGAAGTCTCA GCGAGAATTT 2440
   ATGAGATTTT AAACGCGATC GCTCAATCTA AAGAGAGTGA ATTCCTTATT TTGATTAGCG 2500
   A 2501

(2) INFORMATIONS POUR LA SEQ ID NO: 7:

(i) CARACTERISTIQUES DE LA SEQUENCE:
(A) LONGUEUR: 732 acides aminés
(B) TYPE: acide aminé
(D) CONFIGURATION: linéaire

(ii) TYPE DE MOLECULE: protéine
(xi) DESCRIPTION DE LA SEQUENCE: SEQ ID NO: 7:

Met Ala Asn Glu Arg Ser Lys Leu Ala Phe Lys Lys Thr Phe Pro Val
1               5                   10                  15

Phe Lys Arg Phe Leu Gln Ser Lys Asp Leu Ala Leu Val Val Phe Val
            20                  25                  30

Ile Ala Ile Leu Ala Ile Ile Ile Val Pro Leu Pro Pro Phe Val Leu
        35                  40                  45

Asp Phe Leu Leu Thr Ile Ser Ile Ala Leu Ser Val Leu Ile Ile Leu
    50                  55                  60

Ile Gly Leu Tyr Ile Asp Lys Pro Thr Asp Phe Ser Ala Phe Pro Thr
65                  70                  75                  80

Leu Leu Leu Ile Val Thr Leu Tyr Arg Leu Ala Leu Asn Val Ala Thr
                85                  90                  95

Thr Arg Met Ile Leu Thr Gln Gly Tyr Lys Gly Pro Ser Ala Val Ser
            100                 105                 110

Ile Ile Ile Thr Ala Phe Gly Glu Phe Ser Val Ser Gly Asn Tyr Val
        115                 120                 125

Ile Gly Ala Ile Ile Phe Ser Ile Leu Val Leu Val Asn Leu Leu Val
    130                 135                 140

Val Thr Asn Gly Ser Thr Arg Val Thr Glu Val Arg Ala Arg Phe Ala
145                 150                 155                 160

Leu Asp Ala Met Pro Gly Lys Gln Met Ala Ile Asp Ala Asp Leu Asn
                165                 170                 175

Ser Gly Leu Ile Asp Asp Lys Glu Ala Lys Lys Arg Arg Ala Ala Leu
            180                 185                 190

Ser Gln Glu Ala Asp Phe Tyr Gly Ala Met Asp Gly Ala Ser Lys Phe
        195                 200                 205

Val Lys Gly Asp Ala Ile Ala Ser Ile Ile Ile Thr Leu Ile Asn Ile
    210                 215                 220

Ile Gly Gly Phe Leu Val Gly Val Phe Gln Arg Asp Met Ser Leu Ser
225                 230                 235                 240

Phe Ser Ala Ser Thr Phe Thr Ile Leu Thr Ile Gly Asp Gly Leu Val
                245                 250                 255

Gly Gln Ile Pro Ala Leu Ile Ile Ala Thr Arg Thr Gly Ile Val Ala
            260                 265                 270

Thr Arg Thr Thr Gln Asn Glu Glu Glu Asp Phe Ala Ser Lys Leu Ile
        275                 280                 285

Thr Gln Leu Thr Asn Lys Ser Lys Thr Leu Val Ile Val Gly Ala Ile
    290                 295                 300

Tyr Cys Phe Cys Thr Ile Pro Gly Leu Pro Thr Phe Ser Leu Ala Phe
305                 310                 315                 320

Val Gly Ala Leu Phe Leu Phe Ile Ala Trp Leu Ile Ser Arg Glu Gly
                325                 330                 335

Lys Asp Gly Leu Leu Thr Lys Leu Glu Asn Tyr Leu Ser Gln Lys Phe
            340                 345                 350

Gly Leu Asp Leu Ser Glu Lys Pro His Ser Ser Lys Ile Lys Pro His
        355                 360                 365

Ala Pro Thr Thr Arg Ala Lys Thr Gln Glu Glu Ile Lys Arg Glu Glu
    370                 375                 380

Glu Gln Ala Ile Asp Glu Val Leu Lys Ile Glu Phe Leu Glu Leu Ala
385                 390                 395                 400

Leu Gly Tyr Gln Leu Tyr Ser Leu Ala Asp Met Lys Gln Gly Gly Asp
                405                 410                 415

Leu Leu Glu Arg Ile Arg Gly Ile Arg Lys Lys Ile Ala Ser Asp Tyr
            420                 425                 430

Gly Phe Leu Met Pro Gln Ile Arg Ile Arg Asp Asn Leu Gln Leu Pro
        435                 440                 445

Pro Thr His Tyr Glu Ile Lys Leu Lys Gly Ile Val Ile Gly Glu Gly
    450                 455                 460

Met Val Met Pro Asp Lys Phe Leu Ala Met Asn Thr Gly Phe Val Asn
465                 470                 475                 480

Lys Glu Ile Glu Gly Ile Pro Thr Lys Glu Pro Ala Phe Gly Met Asp
                485                 490                 495

Ala Leu Trp Ile Glu Thr Lys Asn Lys Glu Glu Ala Ile Ile Gln Gly
            500                 505                 510

Tyr Thr Ile Ile Asp Pro Ser Thr Val Ile Ala Thr His Thr Ser Glu
        515                 520                 525

Leu Val Lys Lys Tyr Ala Glu Asp Phe Ile Thr Lys Asp Glu Val Lys
    530                 535                 540

Ser Leu Leu Glu Arg Leu Ala Lys Asp Tyr Pro Thr Ile Val Glu Glu
545                 550                 555                 560

Ser Lys Lys Ile Pro Thr Gly Ala Ile Arg Ser Val Leu Gln Ala Leu
                565                 570                 575

Leu His Glu Lys Ile Pro Ile Lys Asp Met Leu Thr Ile Leu Glu Thr
            580                 585                 590

Ile Thr Asp Ile Ala Pro Leu Val Gln Asn Asp Val Asn Ile Leu Thr
        595                 600                 605

Glu Gln Val Arg Ala Arg Leu Ser Arg Val Ile Thr Asn Ala Phe Lys
    610                 615                 620

Ser Glu Asp Gly Arg Leu Lys Phe Leu Thr Phe Ser Thr Asp Ser Glu
625                 630                 635                 640

Gln Phe Leu Leu Asn Lys Leu Arg Glu Asn Gly Thr Ser Lys Ser Leu
                645                 650                 655

Leu Leu Asn Val Gly Glu Leu Gln Lys Leu Ile Glu Ala Val Ser Glu
            660                 665                 670

Glu Ala Met Lys Val Leu Gln Lys Gly Ile Ala Pro Val Ile Leu Ile
        675                 680                 685

Val Glu Pro Asn Leu Arg Lys Ala Leu Ser Asn Gln Met Glu Gln Ala
    690                 695                 700

Arg Ile Asp Val Ile Val Leu Ser His Ala Glu Leu Asp Pro Asn Ser
705                 710                 715                 720

Asn Phe Glu Ala Leu Gly Thr Ile His Ile Asn Phe
                725                 730

(2) INFORMATIONS POUR LA SEQ ID NO: 8:

(i) CARACTERISTIQUES DE LA SEQUENCE:
(A) LONGUEUR: 724 acides aminés
(B) TYPE: acide aminé
(C) NOMBRE DE BRINS: simple
(D) CONFIGURATION: linéaire

(ii) TYPE DE MOLECULE: protéine

(xi) DESCRIPTION DE LA SEQUENCE: SEQ ID NO: 8:

Met Ala Lys Asn Lys Ile Val Asp Leu Val Phe Pro Phe Leu Gly Pro
1               5                   10                  15

Leu Ile Ala Pro Val Leu Lys Ala Lys Ser Leu Thr Ile Val Gly Phe
            20                  25                  30

Leu Val Cys Ile Leu Ala Ile Ile Ile Val Pro Leu Pro Ser Pro Ile
        35                  40                  45

Leu Asp Phe Phe Leu Ala Leu Ser Ile Ala Leu Ser Val Leu Ile Ile
    50                  55                  60

Leu Ile Ser Ile Tyr Ile Pro Lys Pro Thr Asp Leu Thr Thr Phe Pro
65                  70                  75                  80

Thr Leu Ile Leu Ile Ile Thr Leu Phe Arg Leu Ser Leu Asn Ile Ala
                85                  90                  95

Thr Thr Arg Met Ile Leu Ser Glu Gly Gln Asn Gly Pro Glu Ala Val
            100                 105                 110

Ser Glu Ile Ile Ala Ala Phe Gly Glu Phe Val Val Gly Gly Asn Met
        115                 120                 125

Val Ile Gly Val Ile Val Phe Cys Ile Leu Val Leu Ile Asn Phe Met
    130                 135                 140

Val Val Thr Lys Gly Ser Thr Arg Val Ser Glu Val Gln Ala Arg Phe
145                 150                 155                 160

Thr Leu Asp Ala Met Pro Gly Lys Gln Met Ala Ile Asp Ala Asp Leu
                165                 170                 175

Asn Ala Gly Leu Ile Asp Glu Gln Thr Ala Arg Ala Arg Arg Gln Glu
            180                 185                 190

Val Ile Ala Glu Ala Asn Phe Tyr Gly Ala Met Asp Gly Ser Ser Lys
        195                 200                 205

Phe Ile Lys Gly Asp Ala Val Ala Gly Ile Ile Ile Thr Ile Ile Asn
    210                 215                 220

Ile Ile Gly Gly Phe Leu Ile Gly Ser Phe Gln His Asp Met Ala Leu
225                 230                 235                 240

Ser Asp Ala Ala Ser Thr Tyr Thr Ile Leu Thr Ile Gly Asp Gly Leu
                245                 250                 255

Val Ser Gln Ile Pro Gly Leu Ile Thr Ser Thr Ala Thr Ala Ile Ile
                260                 265                 270

Ile Thr Arg Ala Ser Lys Asp Glu Glu Asn Phe Ala Glu Gly Thr Leu
        275                 280                 285

Thr Gln Leu Leu Ser Glu Tyr Arg Thr Leu Leu Ile Val Gly Phe Val
    290                 295                 300

Leu Phe Ile Phe Ala Leu Val Pro Gly Leu Pro Thr Leu Ser Leu Gly
305                 310                 315                 320

Phe Met Ala Leu Val Phe Leu Ser Leu Gly Tyr Leu Thr Lys Gln Val
                325                 330                 335

Lys Glu Gly Lys Ile Asp Ile Thr Thr Val Lys Lys Ser Lys Pro Ser
            340                 345                 350

Ala Ala Val Ala Ser Gln Ser Gly Ala Gly Gly Thr Thr Ala Ala Pro
        355                 360                 365

Ala Lys Lys Ser Glu Glu Glu Ile Leu Lys Glu Glu Glu His Lys Ile
    370                 375                 380

Asn Asp Ile Leu Lys Val Glu Ile Leu Glu Leu Glu Leu Gly Tyr Gly
385                 390                 395                 400

Leu Ile Lys Leu Ala Glu Asn Glu Leu Thr Glu Arg Ile Arg Ser Met
                405                 410                 415

Arg Arg Ser Ile Ala Glu Ser Leu Gly Phe Leu Met Pro Lys Ile Arg
            420                 425                 430

Ile Arg Asp Asn Leu Arg Leu Lys Pro Asn Glu Tyr Ser Phe Lys Leu
        435                 440                 445

Lys Gly Val Ser Ile Ala Ser Ala Glu Ile Tyr Pro Asp Lys Tyr Leu
    450                 455                 460

Ala Met Asp Ser Gly Phe Ile Thr Glu Glu Ile Glu Gly Ile Ala Thr
465                 470                 475                 480

Lys Glu Pro Ala Phe Asn Ser Asp Ala Leu Trp Ile Asp Ala Asn Leu
                485                 490                 495

Lys Asp Glu Ala Thr Leu Asn Gly Tyr Ile Val Ile Asp Pro Ala Ser
            500                 505                 510

Val Ile Ser Thr His Met Ser Glu Leu Ile Lys Ala His Ala Ser Glu
        515                 520                 525

Leu Leu Thr Arg Gln Glu Val Gln Asn Leu Leu Asp Lys Val Lys Asn
    530                 535                 540

Asp Tyr Pro Ile Ile Val Glu Gly Ala Leu Gly Val Ala Pro Val Ser
545                 550                 555                 560

Leu Ile Gln Lys Ile Leu Lys Asp Leu Leu Lys His His Ile Pro Ile
                565                 570                 575

Lys Asp Met Leu Thr Ile Leu Glu Ser Val Ser Asp Ile Ala Glu Val
            580                 585                 590

Ser Lys Ser Phe Asp Met Ile Ile Glu His Val Arg Ala Ser Leu Ala
        595                 600                 605

Arg Met Ile Thr Asn Met Tyr Leu Asp Asp Lys Gly Asn Leu Asp Ile
    610                 615                 620

Phe Ile Leu Asp Ser Ala Ser Ser Ala Val Leu Met Glu Asn Val Gln
625                 630                 635                 640

Phe Arg Asp Gly Ser Tyr His Leu Pro Leu Ser Val Ala Gln Thr Gly
                645                 650                 655

Thr Leu Val Asp Thr Leu Arg Ala Glu Val Ala Ala Val Ala Asn Gly
            660                 665                 670

Arg Ile Lys Pro Phe Ile Leu Cys Val Glu Pro Gln Leu Arg Lys Phe
        675                 680                 685

Ile Ala Asp Ile Cys Tyr Asn Phe Ser Ile Asn Ile Val Val Leu Ser
    690                 695                 700

Phe Ala Glu Ile Ala Glu Asn Thr Asn Phe Asn Thr Glu Gly Ile Ile
705                 710                 715                 720

Arg Ile Glu Leu

(2) INFORMATIONS POUR LA SEQ ID NO: 9:

    (i) CARACTERISTIQUES DE LA SEQUENCE:
        (A) LONGUEUR: 700 acides aminés
        (B) TYPE: acide aminé
        (C) NOMBRE DE BRINS: simple
        (D) CONFIGURATION: linéaire

    (ii) TYPE DE MOLECULE: protéine

    (xi) DESCRIPTION DE LA SEQUENCE: SEQ ID NO: 9:

Met Ala Asp Ala Ala Ala Pro Asn Ala Ser Ser Met Pro Ser Ala Lys
1               5                   10                  15

Ser Leu Leu Asp Gly Leu Met Arg Gly Glu Met Gly Leu Ala Leu Gly
            20                  25                  30

Val Val Gly Ile Ile Val Leu Leu Ile Ile Pro Val Pro Ala Pro Leu
        35                  40                  45

Leu Asp Val Leu Leu Ala Ile Ser Leu Thr Gly Ser Val Leu Ile Leu
    50                  55                  60

Met Thr Ala Ile Leu Ile Lys Lys Pro Leu Glu Phe Thr Ser Phe Pro
65                  70                  75                  80

Thr Val Leu Leu Val Thr Thr Leu Phe Arg Leu Gly Leu Asn Ile Ala
                85                  90                  95

Ser Thr Arg Leu Ile Leu Ser His Gly Gln Glu Gly Thr Gly Gly Ala
            100                 105                 110

Gly Ala Val Ile Glu Ala Phe Gly His Leu Met Met Gln Gly Asn Phe
        115                 120                 125

Val Ile Gly Val Ile Val Phe Ile Ile Leu Ile Val Val Asn Phe Met
    130                 135                 140

Val Val Thr Lys Gly Ser Gly Arg Ile Ala Glu Val Ala Ala Arg Phe
145                 150                 155                 160

Thr Leu Asp Ser Met Pro Gly Lys Gln Met Ala Ile Asp Ala Asp Leu
                165                 170                 175

Ser Thr Gly Leu Ile Ser Gln Asp Glu Ala Lys Ile Arg Arg Lys Glu
            180                 185                 190

Leu Glu Gln Glu Ser Thr Phe Phe Gly Ala Met Asp Gly Ala Ser Lys
        195                 200                 205

Phe Val Lys Gly Asp Ala Ile Ala Gly Leu Ile Ile Thr Ala Ile Asn
    210                 215                 220

Ile Ile Gly Gly Ile Ile Ile Gly Val Val Gln His Lys Met Pro Phe
225                 230                 235                 240

Gly Asp Ala Ala Ser Thr Tyr Thr Ile Met Thr Ile Gly Asp Gly Leu
                245                 250                 255

Val Ser Gln Ile Pro Ala Leu Ile Ile Ser Ile Ala Ala Gly Met Val
            260                 265                 270

Val Ser Lys Ala Gly Val Glu Gly Ser Ala Asp Lys Ala Leu Thr Thr
        275                 280                 285

Gln Leu Ala Met Asn Pro Val Gly Leu Gly Met Val Ser Ala Ser Ser
    290                 295                 300

Gly Ile Ile Ala Leu Ile Pro Gly Met Pro Ile Phe Pro Phe Ala Ala
305                 310                 315                 320

Met Ala Leu Ala Ala Gly Ala Leu Ala Tyr Lys Arg Val Gln Asp Ala
                325                 330                 335

Lys Lys Pro Lys Ala Leu Asp Pro Ala Asp Leu Glu Ala Ala Ala Pro
            340                 345                 350

Ser Glu Pro Glu Glu Glu Pro Ile Ser Ala Ser Leu Ala Ile Asp Asp
        355                 360                 365

Val Lys Ile Glu Leu Gly Tyr Gly Leu Leu Thr Leu Ile Asn Asp Leu
    370                 375                 380

Asp Gly Arg Lys Leu Thr Asp Gln Ile Arg Ala Leu Arg Lys Thr Leu
385                 390                 395                 400

Ala Ser Glu Tyr Gly Phe Val Met Pro Pro Val Arg Ile Leu Asp Asn
                405                 410                 415

Met Arg Leu Ala Asn Gln Gly Tyr Ala Ile Arg Ile Lys Glu Met Glu
            420                 425                 430

Ala Gly Ala Gly Glu Val Arg Leu Gly Cys Leu Met Cys Met Asp Pro
        435                 440                 445

Arg Gly Gly Gln Val Glu Leu Pro Gly Glu His Val Arg Glu Pro Ala
    450                 455                 460

Phe Gly Leu Pro Ala Thr Trp Ile Ala Asp Asp Leu Arg Glu Glu Ala
465                 470                 475                 480

Thr Phe Arg Gly Tyr Thr Val Val Asp Pro Ala Thr Val Leu Thr Thr
                485                 490                 495

His Leu Thr Glu Ile Leu Lys Glu Asn Met Ala Asp Leu Leu Ser Tyr
            500                 505                 510

Ala Glu Val Gln Lys Leu Leu Lys Glu Leu Pro Glu Thr Gln Lys Lys
        515                 520                 525

Leu Val Asp Asp Leu Ile Pro Gly Thr Val Thr Ala Thr Thr Val Gln
    530                 535                 540

Arg Val Leu Gln Ser Leu Leu Arg Glu Arg Val Ser Ile Arg Asp Leu
545                 550                 555                 560

Pro Gln Ile Leu Glu Gly Val Gly Glu Ala Ala Pro His Thr Ala Ser
                565                 570                 575

Val Thr Gln Leu Val Glu Gln Val Arg Ala Arg Leu Ala Arg Gln Leu
            580                 585                 590

Cys Trp Ala Asn Arg Gly Asp Asp Gly Ala Leu Pro Ile Ile Thr Leu
        595                 600                 605

Ser Ala Asp Trp Glu Gln Ala Phe Ala Glu Ala Leu Ile Gly Pro Gly
    610                 615                 620

Asp Asp Lys Gln Leu Ala Leu Pro Pro Ser Arg Leu Gln Asp Phe Ile
625                 630                 635                 640

Arg Gly Val Arg Asp Ser Phe Glu Arg Ala Ala Leu Ala Gly Glu Ala
                645                 650                 655

Pro Val Leu Leu Thr Ser Pro Gly Val Arg Pro Tyr Val Arg Ser Ile
            660                 665                 670

Ile Glu Arg Phe Arg Gly Gln Thr Val Val Met Ser Gln Asn Glu Ile
        675                 680                 685

His Pro Arg Ala Arg Leu Lys Thr Val Gly Met Val
    690                 695                 700

(2) INFORMATIONS POUR LA SEQ ID NO: 10:

    (i) CARACTERISTIQUES DE LA SEQUENCE:
        (A) LONGUEUR: 704 acides aminés
        (B) TYPE: acide aminé
        (C) NOMBRE DE BRINS: simple
        (D) CONFIGURATION: linéaire

   (ii) TYPE DE MOLECULE: protéine

(xi) DESCRIPTION DE LA SEQUENCE: SEQ ID NO: 10:

Met Asn Pro His Asp Leu Glu Trp Leu Asn Arg Ile Gly Glu Arg Lys
1               5                   10                  15

Asp Ile Met Leu Ala Val Leu Leu Leu Ala Val Val Phe Met Met Val
            20                  25                  30

Leu Pro Leu Pro Pro Leu Val Leu Asp Ile Leu Ile Ala Val Asn Met
        35                  40                  45

Thr Ile Ser Val Val Leu Leu Met Ile Ala Ile Tyr Ile Asn Ser Pro
    50                  55                  60

Leu Gln Phe Ser Ala Phe Pro Ala Val Leu Leu Val Thr Thr Leu Phe
65                  70                  75                  80

Arg Leu Ala Leu Ser Val Ser Thr Thr Arg Met Ile Leu Leu Gln Ala
                85                  90                  95

Asp Ala Gly Gln Ile Val Tyr Thr Phe Gly Asn Phe Val Val Gly Gly
            100                 105                 110

Asn Leu Ile Val Gly Ile Val Ile Phe Leu Ile Ile Thr Ile Val Gln
        115                 120                 125

Phe Leu Val Ile Thr Lys Gly Ser Glu Arg Val Ala Glu Val Ser Ala
    130                 135                 140

Arg Phe Ser Leu Asp Ala Met Pro Gly Lys Gln Met Ser Ile Asp Gly
145                 150                 155                 160

Asp Met Arg Ala Gly Val Ile Asp Val Asn Glu Ala Arg Glu Arg Arg
                165                 170                 175

Ala Thr Ile Glu Lys Glu Ser Gln Met Phe Gly Ser Met Asp Gly Ala
            180                 185                 190

Met Lys Phe Val Lys Gly Asp Ala Ile Ala Gly Leu Ile Ile Ile Phe
        195                 200                 205

Val Asn Ile Leu Gly Gly Val Thr Ile Gly Val Thr Gln Lys Gly Leu
    210                 215                 220

Ala Ala Ala Glu Ala Leu Gln Leu Tyr Ser Ile Leu Thr Val Gly Asp
225                 230                 235                 240

Gly Met Val Ser Gln Val Pro Ala Leu Leu Ile Ala Ile Thr Ala Gly
                245                 250                 255

Ile Ile Val Thr Arg Val Ser Ser Glu Asp Ser Ser Asp Leu Gly Ser
            260                 265                 270

Asp Ile Gly Lys Gln Val Val Ala Gln Pro Lys Ala Met Leu Ile Gly
        275                 280                 285

Gly Val Leu Leu Leu Leu Phe Gly Leu Ile Pro Gly Phe Pro Thr Val
    290                 295                 300

Thr Phe Leu Ile Leu Ala Leu Leu Val Gly Cys Gly Gly Tyr Met Leu
305                 310                 315                 320

Ser Arg Lys Gln Ser Arg Asn Asp Glu Ala Asn Gln Asp Leu Gln Ser
                325                 330                 335

Ile Leu Thr Ser Gly Ser Gly Ala Pro Ala Ala Arg Thr Lys Ala Lys
            340                 345                 350

Thr Ser Gly Ala Asn Lys Gly Arg Leu Gly Glu Gln Glu Ala Phe Ala
        355                 360                 365

Met Thr Val Pro Leu Leu Ile Asp Val Asp Ser Ser Gln Gln Glu Ala
    370                 375                 380

Leu Glu Ala Asn Ala Leu Asn Asp Glu Leu Val Arg Val Arg Arg Ala
385                 390                 395                 400

Leu Tyr Leu Asp Leu Gly Val Pro Phe Pro Gly Ile His Leu Arg Phe
                405                 410                 415

Asn Glu Gly Met Gly Glu Gly Glu Tyr Ile Ile Ser Leu Gln Glu Val
            420                 425                 430

Pro Val Ala Arg Gly Glu Leu Lys Ala Gly Tyr Leu Leu Val Arg Glu
        435                 440                 445

Ser Val Ser Gln Leu Glu Leu Leu Gly Ile Pro Tyr Glu Lys Gly Glu
    450                 455                 460

His Leu Leu Pro Asp Gln Glu Ala Phe Trp Val Ser Val Glu Tyr Glu
465                 470                 475                 480

Glu Arg Leu Glu Lys Ser Gln Leu Glu Phe Phe Ser His Ser Gln Val
                485                 490                 495

Leu Thr Trp His Leu Ser His Val Leu Arg Glu Tyr Ala Glu Asp Phe
            500                 505                 510

Ile Gly Ile Gln Glu Thr Arg Tyr Leu Leu Glu Gln Met Glu Gly Gly
        515                 520                 525

Tyr Gly Glu Leu Ile Lys Glu Val Gln Arg Ile Val Pro Leu Gln Arg
    530                 535                 540

Met Thr Glu Ile Leu Gln Arg Leu Val Gly Glu Asp Ile Ser Ile Arg
545                 550                 555                 560

Asn Met Arg Ser Ile Leu Glu Ala Met Val Glu Trp Gly Gln Lys Glu
                565                 570                 575

Lys Asp Val Val Gln Leu Thr Glu Tyr Ile Arg Ser Ser Leu Lys Arg
            580                 585                 590

Tyr Ile Cys Tyr Lys Tyr Ala Asn Gly Asn Asn Ile Leu Pro Ala Tyr
        595                 600                 605

Leu Phe Asp Gln Glu Val Glu Glu Lys Ile Arg Ser Gly Val Arg Gln
    610                 615                 620

Thr Ser Ala Gly Ser Tyr Leu Ala Leu Glu Pro Ala Val Thr Glu Ser
625                 630                 635                 640

Leu Leu Glu Gln Val Arg Lys Thr Ile Gly Asp Leu Ser Gln Ile Gln
                645                 650                 655

Ser Lys Pro Val Leu Ile Val Ser Met Asp Ile Arg Arg Tyr Val Arg
            660                 665                 670

Lys Leu Ile Glu Ser Glu Tyr Tyr Gly Leu Pro Val Leu Ser Tyr Gln
        675                 680                 685

Glu Leu Thr Gln Gln Ile Asn Ile Gln Pro Leu Gly Arg Ile Cys Leu
    690                 695                 700

(2) INFORMATIONS POUR LA SEQ ID NO: 11:

(i) CARACTERISTIQUES DE LA SEQUENCE:
(A) LONGUEUR: 685 acides aminés
(B) TYPE: acide aminé
(C) NOMBRE DE BRINS: simple
(D) CONFIGURATION: linéaire

(ii) TYPE DE MOLECULE: protéine

(xi) DESCRIPTION DE LA SEQUENCE: SEQ ID NO: 11:

Met Leu Leu Ser Leu Leu Asn Ser Ala Arg Leu Arg Pro Glu Leu Leu
1               5                   10                  15

Ile Leu Val Leu Met Val Met Ile Ile Ser Met Phe Val Ile Pro Leu
            20                  25                  30

Pro Thr Tyr Leu Val Asp Phe Leu Ile Ala Leu Asn Ile Val Leu Ala
        35                  40                  45

Ile Leu Val Phe Met Gly Ser Phe Tyr Ile Asp Arg Ile Leu Ser Phe
    50                  55                  60

Ser Thr Phe Pro Ala Val Leu Leu Ile Thr Thr Leu Phe Arg Leu Ala
65                  70                  75                  80

Leu Ser Ile Ser Thr Ser Arg Leu Ile Leu Ile Glu Ala Asp Ala Gly
                85                  90                  95

Glu Ile Ile Ala Thr Phe Gly Gln Phe Val Ile Gly Asp Ser Leu Ala
            100                 105                 110

Val Gly Phe Val Val Phe Ser Ile Val Thr Val Val Gln Phe Ile Val
        115                 120                 125

Ile Thr Lys Gly Ser Glu Arg Val Ala Glu Val Ala Ala Arg Phe Ser
    130                 135                 140

Leu Asp Gly Met Pro Gly Lys Gln Met Ser Ile Asp Ala Asp Leu Lys
145                 150                 155                 160

Ala Gly Ile Ile Asp Ala Asp Ala Ala Arg Glu Arg Arg Ser Val Leu
                165                 170                 175

Glu Arg Glu Ser Gln Leu Tyr Gly Ser Phe Asp Gly Ala Met Lys Phe
            180                 185                 190

Ile Lys Gly Asp Ala Ile Ala Gly Ile Ile Ile Phe Val Asn Phe
        195                 200                 205

Ile Gly Gly Ile Ser Val Gly Met Thr Arg His Gly Met Asp Leu Ser
    210                 215                 220

Ser Ala Leu Ser Thr Tyr Thr Met Leu Thr Ile Gly Asp Gly Leu Val
225                 230                 235                 240

Ala Gln Ile Pro Ala Leu Leu Ile Ala Ile Ser Ala Gly Phe Ile Val
                245                 250                 255

Thr Arg Val Asn Gly Asp Thr Asp Asn Met Gly Arg Asn Ile Met Thr
            260                 265                 270

Gln Leu Leu Asn Asn Pro Phe Val Leu Val Val Thr Ala Ile Leu Thr
        275                 280                 285

Ile Ser Met Gly Thr Leu Pro Gly Phe Pro Leu Pro Val Phe Val Ile
    290                 295                 300

Leu Ser Val Val Leu Ser Val Leu Phe Tyr Phe Lys Phe Arg Glu Ala
305                 310                 315                 320

Lys Arg Ser Ala Ala Lys Pro Lys Thr Ser Lys Gly Glu Gln Pro Leu
                325                 330                 335

Ser Ile Glu Glu Lys Glu Gly Ser Ser Leu Gly Leu Ile Gly Asp Leu
            340                 345                 350

Asp Lys Val Ser Thr Glu Thr Val Pro Leu Ile Leu Leu Val Pro Lys
        355                 360                 365

Ser Arg Arg Glu Asp Leu Glu Lys Ala Gln Leu Ala Glu Arg Leu Arg
    370                 375                 380

Ser Gln Phe Phe Ile Asp Tyr Gly Val Arg Leu Pro Glu Val Leu Leu
385                 390                 395                 400

Arg Asp Gly Glu Gly Leu Asp Asp Asn Ser Ile Val Leu Leu Ile Asn
                405                 410                 415

Glu Ile Arg Val Glu Gln Phe Thr Val Tyr Phe Asp Leu Met Arg Val
            420                 425                 430

Val Asn Tyr Ser Asp Glu Val Val Ser Phe Gly Ile Asn Pro Thr Ile
        435                 440                 445

His Gln Gln Gly Ser Ser Gln Tyr Phe Trp Val Thr His Glu Glu Gly
    450                 455                 460

Glu Lys Leu Arg Glu Leu Gly Tyr Val Leu Arg Asn Ala Leu Asp Glu
465                 470                 475                 480

Leu Tyr His Cys Leu Ala Val Thr Val Ala Arg Asn Val Asn Glu Tyr
                485                 490                 495

Phe Gly Ile Gln Glu Thr Lys His Met Leu Asp Gln Leu Glu Ala Lys
            500                 505                 510

Phe Pro Asp Leu Leu Lys Glu Val Leu Arg His Ala Thr Val Gln Arg
        515                 520                 525

Ile Ser Glu Val Leu Gln Arg Leu Leu Ser Glu Arg Val Ser Val Arg
    530                 535                 540

Asn Met Lys Leu Ile Met Glu Ala Leu Ala Leu Trp Ala Pro Arg Glu
545                 550                 555                 560

Lys Asp Val Ile Asn Leu Val Glu His Ile Arg Gly Ala Met Ala Arg
                565                 570                 575

Tyr Ile Cys His Lys Phe Ala Asn Gly Gly Glu Leu Arg Ala Val Met
            580                 585                 590

Val Ser Ala Glu Val Glu Asp Val Ile Arg Lys Gly Ile Arg Gln Thr
        595                 600                 605

Ser Gly Ser Thr Phe Leu Ser Leu Asp Pro Glu Ala Ser Ala Asn Leu
    610                 615                 620

Met Asp Leu Ile Thr Leu Lys Leu Asp Asp Leu Leu Ile Ala His Lys
625                 630                 635                 640

Asp Leu Val Leu Leu Thr Ser Val Asp Val Arg Arg Phe Ile Lys Lys
                645                 650                 655

Met Ile Glu Gly Arg Phe Pro Asp Leu Glu Val Leu Ser Phe Gly Glu
            660                 665                 670

Ile Ala Asp Ser Lys Ser Val Asn Val Ile Lys Thr Ile
        675                 680                 685

(2) INFORMATIONS POUR LA SEQ ID NO: 12:

    (i) CARACTERISTIQUES DE LA SEQUENCE:
        (A) LONGUEUR: 666 acides aminés
        (B) TYPE: acide aminé
        (C) NOMBRE DE BRINS: simple
        (D) CONFIGURATION: linéaire

    (ii) TYPE DE MOLECULE: protéine

    (xi) DESCRIPTION DE LA SEQUENCE: SEQ ID NO: 12:

Met Val Met Ile Ile Ala Met Leu Ile Ile Pro Leu Pro Thr Tyr Leu
1               5                   10                  15

Val Asp Phe Leu Ile Gly Leu Asn Ile Val Leu Ala Ile Leu Val Phe
            20                  25                  30

Met Gly Ser Phe Tyr Ile Glu Arg Ile Leu Ser Phe Ser Thr Phe Pro
        35                  40                  45

Ser Val Leu Leu Ile Thr Thr Leu Phe Arg Leu Ala Leu Ser Ile Ser
    50                  55                  60

Thr Ser Arg Leu Ile Leu Val Asp Ala Asp Arg Gly Lys Ile Ile Thr
65                  70                  75                  80

Thr Phe Gly Gln Phe Val Ile Gly Asp Ser Leu Ala Val Gly Phe Val
                85                  90                  95

Ile Phe Ser Ile Val Thr Val Val Gln Phe Ile Val Ile Thr Lys Gly
            100                 105                 110

Ser Glu Arg Val Ala Glu Val Ala Ala Arg Phe Ser Leu Asp Gly Met
        115                 120                 125

Pro Gly Lys Gln Met Ser Ile Asp Ala Asp Leu Lys Ala Gly Ile Ile
    130                 135                 140

Asp Ala Ala Gly Ala Lys Glu Arg Arg Ser Ile Leu Glu Arg Glu Ser
145                 150                 155                 160

Gln Leu Tyr Gly Ser Phe Asp Gly Ala Met Lys Phe Ile Lys Gly Asp
                165                 170                 175

Ala Ile Ala Gly Ile Ile Ile Ile Phe Val Asn Leu Ile Gly Gly Ile
            180                 185                 190

Ser Val Gly Met Ser Gln His Gly Met Ser Leu Ser Gly Ala Leu Ser
        195                 200                 205

Thr Tyr Thr Ile Leu Thr Ile Gly Asp Gly Leu Val Ser Gln Ile Pro
    210                 215                 220

Ala Leu Leu Ile Ser Ile Ser Ala Gly Phe Met Leu Thr Arg Val Asn
225                 230                 235                 240

Gly Asp Ser Asp Asn Met Gly Arg Asn Ile Met Ser Gln Ile Phe Gly
                245                 250                 255

Asn Pro Phe Val Leu Ile Val Thr Ser Ala Leu Ala Leu Ala Ile Gly
            260                 265                 270

Met Leu Pro Gly Phe Pro Phe Phe Val Phe Phe Leu Ile Ala Val Thr
        275                 280                 285

Leu Thr Ala Leu Phe Tyr Tyr Lys Lys Val Val Glu Lys Glu Lys Ser
    290                 295                 300

Leu Ser Glu Ser Asp Ser Ser Gly Tyr Thr Gly Thr Phe Asp Ile Asp
305                 310                 315                 320

Asn Thr His Asp Ser Ser Leu Ala Met Ile Glu Asn Leu Asp Arg Ile
                325                 330                 335

Ser Ser Glu Thr Val Pro Leu Ile Leu Leu Phe Ala Glu Asn Lys Ile
            340                 345                 350

Asn Ala Asn Asp Met Glu Gly Leu Ile Glu Arg Ile Arg Ser Gln Phe
        355                 360                 365

Phe Ile Asp Tyr Gly Val Arg Leu Pro Thr Ile Leu Tyr Arg Thr Ser
    370                 375                 380

Asn Glu Leu Lys Val Asp Asp Ile Val Leu Leu Ile Asn Glu Val Arg
385                 390                 395                 400

Ala Asp Ser Phe Asn Ile Tyr Phe Asp Lys Val Cys Ile Thr Asp Glu
                405                 410                 415

Asn Gly Asp Ile Asp Ala Leu Gly Ile Pro Val Val Ser Thr Ser Tyr
            420                 425                 430

Asn Glu Arg Val Ile Ser Trp Val Asp Val Ser Tyr Thr Glu Asn Leu
        435                 440                 445

Thr Asn Ile Asp Ala Lys Ile Lys Ser Ala Gln Asp Glu Phe Tyr His
    450                 455                 460

Gln Leu Ser Gln Ala Leu Leu Asn Asn Ile Asn Glu Ile Phe Gly Ile
465                 470                 475                 480

Gln Glu Thr Lys Asn Met Leu Asp Gln Phe Glu Asn Arg Tyr Pro Asp
                485                 490                 495

Leu Leu Lys Glu Val Phe Arg His Val Thr Ile Gln Arg Ile Ser Glu
            500                 505                 510

Val Leu Gln Arg Leu Leu Gly Glu Asn Ile Ser Val Arg Asn Leu Lys
        515                 520                 525

Leu Ile Met Glu Ser Leu Ala Leu Trp Ala Pro Arg Glu Lys Asp Val
    530                 535                 540

Ile Thr Leu Val Glu His Val Arg Ala Ser Leu Ser Arg Tyr Ile Cys
545                 550                 555                 560

Ser Lys Ile Ala Val Ser Gly Glu Ile Lys Val Val Met Leu Ser Gly
                565                 570                 575

Tyr Ile Glu Asp Ala Ile Arg Lys Gly Ile Arg Gln Thr Ser Gly Gly
            580                 585                 590

Ser Phe Leu Asn Met Asp Ile Glu Val Ser Asp Glu Val Met Glu Thr
        595                 600                 605

Leu Ala His Ala Leu Arg Glu Leu Arg Asn Ala Lys Lys Asn Phe Val
    610                 615                 620

Leu Leu Val Ser Val Asp Ile Arg Arg Phe Val Lys Arg Leu Ile Asp
625                 630                 635                 640

Asn Arg Phe Lys Ser Ile Leu Val Ile Ser Tyr Ala Glu Ile Asp Glu
                645                 650                 655

Ala Tyr Thr Ile Asn Val Leu Lys Thr Ile
            660                 665